# EUROPEAN PATENT APPLICATION

(11) **EP 4 791 032 A1**
(43) Date of publication of application: **12.08.2026**
(21) Application number: 25156466.2
(22) Date of filing: 07.02.2025
(51) Int. Cl.: H04R 1/10, A61F 11/06, A61F 11/14, H04M 1/18, H04W 4/10, H04R 5/033, H01H 9/02

(54) **HEARING PROTECTION AND COMMUNICATION SYSTEM WITH CONFIGURABLE TANGIBLE CONTROLS**

(71) Applicant: INVISIO A/S, 2650 Hvidovre (DK)
(72) Inventor: PETERSEN, Jacob, 2650 Hvidovre (DK); GRÜNEWALD, Manuel, 2650 Hvidovre (DK); BODÉN, Richard, 2650 Hvidovre (DK); DAHL, Jonas Burup, 2650 Hvidovre (DK); PEDERSEN, Sine Storm, 2650 Hvidovre (DK); ANDERSEN, Mathias Bach, 2650 Hvidovre (DK); BONNELYKKE, Jesper Rye, 2650 Hvidovre (DK)
(74) Representative: Guardian IP Consulting I/S

(57) **Abstract**

A communication system for communicating in a harsh environment includes hearing protection headsets with left and right earcups to attenuate external sounds. The communication module connects with both a first and second type of peripheral communication device, receiving and transmitting audio signals and control signals to the speakers in the earcups and the peripheral devices. The user interface comprises tangible controls that enable the communication module to transmit control instructions to both types of peripheral devices using distinct control signals.

## Description

### TECHNICAL FIELD

The present disclosure relates to a hearing protection and communication system with configurable tangible controls to be used in a harsh and demanding environment.

### BACKGROUND

Communication and hearing protection systems are essential for professionals operating in noisy and mission critical environments to work safely and effectively while protecting their hearing. Clear and undistorted communication is vital for military and public safety professionals operating in harsh and demanding environments. Clear and undistorted communication is essential for soldiers, police, rescue personnel, fire fighters, and other task forces as it facilitates improved coordination among team members and enhances safety and security by reducing misunderstandings. Rapid and swift communication facilitates quick decision-making and responses to changing situations. Besides communication capabilities, it is utmost important to shield individuals who requires to operate in harsh environments against exposure to harmful external noise levels such as vehicle noise, gun shots, explosions, etc.

Hearing protection devices are generally known and used amount soldiers, police forces etc. for noise attenuation. Typically, passive hearing protecting devices such as foam earplugs or earmuffs may be used to physically blocking or dampening sound waves thereby protecting the user against harmful high noise exposure; however this type of hearing protecting devices blocks out all type of sound, which can be extremely problematic for many types of operations, where the user is required to maintain both situation awareness of the surroundings and clear communication with other team members via radio frequency (RF) communication connection. Thus, for personal operating in harsh and demanding environments with high noise exposure, it is essential to wear communication equipment allowing for both situational awareness, clear communication and hearing protection.

The communication equipment utilized by military and public safety professionals across different agencies, units and teams may span widely from older analog RF units to new modern digital systems and devices, which may require modern hearing protecting and communication devices to be capable of interfacing both legacy communication systems as well as newer digital communication devices. Thus, in one situation there is a need to develop a hearing protection and communication system to be worn by a user in harsh environments capable of interfacing and controlling a wide range of communication devices.

### SUMMARY

One object of the present disclosure is to overcome at least some of the above-mentioned and/or other disadvantages (at least to an extent), or at least to provide an alternative to existing solutions.

The present disclosure provides embodiments of a hearing protection and communication system with configurable tangible controls to be used in a harsh and demanding environment as disclosed herein according to independent claims with advantageous embodiments as defined by the dependent claims and disclosed herein.

According to a first aspect, the present disclosure provides: a communication system for communicating in a harsh environment, comprising: a hearing protection headset to be worn by a user in the harsh environment, said headset comprising: a left and a right earcup configured to attenuate external sounds from the harsh environment.

The communication system further comprises a communication module configured to:
connect with a first type of peripheral communication device and a second type of peripheral communication device,
- receive at least one audio signal from the first type of peripheral communication device and/or the second type of peripheral communication device,
- transmit the at least one audio signal to a speaker in the left earcup and/or a speaker in the right earcup,
- transmit a first control-signal comprising one or more first control instruction(s) of and to the first type of peripheral communication device, and
- transmit a second control-signal comprising one or more second control instruction(s) of and to the second type of peripheral communication device,
- wherein the first control-signal is a first type of signal, and the second control-signal is a second type of signal that differs from the first type of signal.

By providing the first control-signal being a first type of signal, and the second control-signal being a second type of signal that differs from the first type of signal, it is possible to control the first type of peripheral communication device and the second type of peripheral communication device, particularly in a situation where the first type of peripheral communication device is different from the second type of peripheral communication device, for example where the first type of peripheral communication device is configured to be controlled by a first type of signal in the form of an analog signal, and where the second type of peripheral communication device is configured to be controlled by a second type of signal in the form of a digital signal. This may be particularly advantageous for personal operating in harsh and demanding environments as peripheral communication devices utilized by military and public safety professionals across different agencies, units and teams may spans widely from a first type such as older analog RF Land Mobile Radios (LMR) to a second type such as a newer modern digital radios and communication devices like a Mobile Ad Hoc Networking (MANET) radio, tactical smartphone (EUD) or a multiple-input and multiple-output (MIMO) radio etc. The communication system according to the first aspect provides a modern hearing protecting and communication system capable of interfacing and communicating with both legacy analog communication devices as well as newer digital communication devices all being part of the available equipment in operation across military and public safety units around the world for establishing clear and undistorted communication in harsh and demanding environments. Certain situations may for example arise in a harsh environment. For example users may be required to swap between a first- and second type of peripheral communication during a mission or in-between tasks or assignments, making the communication system according to the present disclosure highly desirable as being able to transmit or receive the correct voice communication in a complex communication setup, independent of communication device types and under stressful circumstances, can make the difference between life and death.

In a preferred embodiment of the first aspect, the communication system further comprises a user interface in contact with the communication module.

In this way, a user may physically interact with the communication system to provide user input to the communication module, which is advantageous as the user wearing the communication system in harsh environment, may be enabled to provide instructions to the communication module via the user interface

In another preferred embodiment, the user interface comprises one or more tangible controls that are manually operably by the user, wherein each of said tangible controls has at least one operation state.

It may be particular advantageous to have tangible controls on the user interface when used by personal in harsh environments, as the tangible controls should be manually operable by the user under difficult and stressful circumstances to enable clear and undistorted communications, even while wearing thick gloves or being in darkness such that the structure and design of the controls are tangible to enable unambiguously manual operation. Each of the tangible controls may support one or more states of operation, such as single press, double press, long press etc., thereby enabling the user to interact with a tangible control in multiple different way.

In some embodiments each operation state is automatically configured to enable the communication module to:
- transmit one or more control-instruction(s) of said first control-instruction(s) using the first control-signal; or
- transmit one or more control-instruction(s) of said second control-instruction(s) using the second control-signal.

It is particularly advantageous, that the user interface may be configured to enable the communication module to transmit control-instructions to a first- and second type peripheral communication device, as the user may remotely (e.g. via the user interface) control the connected peripheral communication device independently of its type (i.e. both a first type and second type). Personal operation in harsh environment may typically carry a load of personal equipment such as helmet, uniform, boots, armor, weapon(s), ammunition, food, canteens, compass, first aid kit and communication devices, etc. placed strategically on the uniform such that the different effect are easy to reach. The front part of a soldier is typically a crowded space, and the most critical equipment is prioritized, such as ammunition. Communication devices, such as radios, which may vary a lot in weight and form factor, are typically located out of immediate reach for the soldier, such as on the back making remote operation of the communication device highly desirable.

The user interface according to the present disclosure enables the user to provide control-signals for peripheral communication device, such that the user may control communication without directly interacting with the peripheral communication device itself, thereby enabling fast and efficient operation while enabling the soldier to position the peripheral communication device away from the front part of the body, making space for life critical equipment.

By providing that each operation state is automatically defined for each of said tangible controls, there is provided efficient flexibility in terms of functionality for each of said tangible controls. Each of said tangible controls may for example have different functionally dependent on which peripheral communication device type that is desired to be controlled. For example, in one situation, if desired to control a analog communication device, a push on one of said tangible controls may automatically be configured to control (e.g. via the communication module) the first type of peripheral communication device in the form of the analog radio, such that such that the push on the one of said tangible control sends at least one control-instruction using a first control-signal, such as a Push-To-Talk (PTT) signal to the analog radio device via the input line in the transceiver, thereby switching an RF transmitter module on in the radio to make a radio-call. In another example, if the analog radio or replaced with digital device, it desired to control the digital device (e.g. A second type of peripheral communication device). A push on the same tangible control may now be automatically configured to control the second type of peripheral communication device. The pushes on the one of said tangible control may thus send other control-instructions to the digital device, such as for example to make a phone call or transmit a digital image, video or other data using a digital type of control signal (e.g. second type of control signal).

The automatic configuration of said tangible controls may provide a rapid setup of said tangible controls such that the user may not need to define how said tangible controls should interact with the desired peripheral device. In this manner, the user may be less stressed and may allow the user to focus more on the harsh environment rather than on the setup of tangible controls and are thereby enabled to establish clear and undistorted communication swift and easily while maintaining focus on the surroundings. Additionally, the automatic configuration allows the user to swap between peripheral communication device types without changing the hearing protection and communication system and have instantaneous control of the peripheral communication device regardless of its type without cumbersome manual configuration, thereby providing the user clear and undistorted communication independent of device type.

In a preferred embodiment related to the first aspect, the communication module and the user interface are integrated into a single communication device located in the hearing protection headset or located in a head-worn device comprising the hearing protection headset.

In this embodiment, the tactical hearing protection headset contains both the user interface and the communication module, enabling the headset to become a compact standalone unit capable of directly interfacing and control different types of peripheral communication devices directly via tangible controls on the headset. This is particularly advantageous for operating in harsh and demanding environments, as it reduces the required number of devices to be worn by the user, -Accordingly, it may free up more space for the user to carry other essential equipment such as, magazines for ammunition, food and first aid supply, power supplies etc. required by modern military or public safety personal.

A second aspect of the disclosure provides: a communication system for communicating in a harsh environment, comprising: a communication system configured to:
- connect with a first type of peripheral communication device and a second type of peripheral communication device,
- receive at least one audio signal from the first type of peripheral communication device and/or the second type of peripheral communication device,
- transmit the at least one audio signal to a speaker in the left earcup and/or a speaker in the right earcup,
- transmit a first control-signal comprising one or more first control instruction(s) of and to the first type of peripheral communication device, and
- transmit a second control-signal comprising one or more second control instruction(s) of and to the second type of peripheral communication device,
wherein the first control-signal is a first type of signal, and the second control-signal is a second type of signal that differs from the first type of signal.

In a preferred embodiment of the second aspect, the communication system further comprises a user interface in contact with the communication module, wherein the user interface comprises one or more tangible controls that are manually operably by the user, wherein each of said tangible controls has at least one operation state.

In some embodiments each operation state is automatically configured to enable the communication module to:
- transmit one or more control-instruction(s) of said first control-instruction(s) using the first control-signal; or
- transmit one or more control-instruction(s) of said second control-instruction(s) using the second control-signal.

In a preferred embodiment of the second aspect, the communication system further comprises a user interface in contact with the communication module, wherein the user interface comprises one or more tangible controls that are manually operably by the user, wherein each of said tangible controls has at least one operation state.

In some embodiments each operation state is automatically configured to enable the communication module to:
- transmit one or more control-instruction(s) of said first control-instruction(s) using the first control-signal; or
- transmit one or more control-instruction(s) of said second control-instruction(s) using the second control-signal.

In a preferred embodiment the second aspect is part of the first aspect.

The second aspect of the present disclosure provides similar advantages and benefits as described in relation to the first aspect and the aforementioned embodiments thereof.

In a preferred embodiment according to the second aspect, the communication module and the user interface are integrated into a single communication device that is located separately from the hearing protection headset.

In some embodiments, each operation state is automatically configured for each of the tangible controls to enable the communication module (106) to:
- transmit the one or more control-instruction(s) of said first control-instruction(s) using the first control-signal, when the communication module receives first coupling data associated with the first type of peripheral communication device, or
- transmit the one or more control-instruction(s) of said second control-instruction(s) of the second type of peripheral communication device using the second control-signal when the communication module (106) receives second coupling data associated with the second type of peripheral communication device.

It may be particularly advantageous that each operation state is automatically configured to enable the communication module to control a first type of peripheral communication device or a second type of peripheral communication device in response to receiving coupling data associated with the device type. Particularly then the communication module receives first type of coupling data when a first type of peripheral communication device is connected and receives second coupling data when a second type of peripheral communication device is connected. This may allow the user to freely swap between peripheral communication device types connected to the communication system, such as an analog radio or a digital device and maintain instantaneous control of the peripheral communication device regardless of its type without cumbersome manual configuration. In one example, the coupling data associated with the device type may automatically configure one or more of the user interface controls to enable the communication module to communicate and control connected first type or second type of peripheral communication device, thereby providing the user with clear and undistorted communication independent of device type.

In some embodiments, the communication module is configured to receive the first coupling data when the communication module is in contact with the first type of peripheral communication device, and/or wherein the communication module is configured to receive the second coupling data when the communication module is in contact with second type of peripheral communication device.

In some embodiments, the communication module is further configured to receive the first coupling data and/or second coupling data from a socket located in the communication module. Preferably where the socket is configured to recognize the type of device being connected to the socket.

In some embodiments, the communication module is further configured to receive the first coupling data and/or second coupling data from a plug being connected to the communication module. Preferably where the plug comprises a microchip comprising the first coupling data or the second coupling data

Advantageously a plug comprises the first coupling data is connected to a first type of peripheral communication device and a plug comprising the second coupling data is connected to a first type of peripheral communication device. This way, the plug may be directly attached to the peripheral communication device, and upon connection to the communication module, coupling data is obtained via the plug thereby enabling the communication module to recognize the device type.

In some embodiments, the first coupling data comprises said first control-instruction(s), and/or wherein the second coupling data comprises said second control-instruction(s).

In some embodiments, the one or more control-instructions of said first control-instruction(s) is defined according to a first communication protocol or wherein the one or more control-instructions of said second control-instruction(s) is defined according to a second communication protocol.

In some embodiments, the one or more control-instructions of said first control-instruction(s) is defined to control a transceiver-module of the first type of peripheral communication device to establish a person-to-person voice communication.

In a related embodiment, the one or more control-instructions of said second control-instruction(s) is defined to control a transceiver-module of the second type of peripheral communication device to establish a person-to-person voice communication.

In some embodiments, the first control-signal is a digital signal and wherein the second control-signal is an analog signal, or wherein the first control-signal is an analog signal and wherein the second control-signal is a digital signal.

In some embodiments, the analog signal comprises a Push-to-Talk (PTT) signal, preferably where the PTT signal is a DC voltage signal or an AC voltage signal.

In some embodiments, the digital signal comprises digital data packages, preferably where the digital signal is a USB signal, a UART signal, an ethernet signal or a DECT signal

In some embodiments, the at least one operation state of each of said tangible controls is one of:
- a short-press state, wherein the short-press state is defined as an interaction with the tactile control for less than 200 ms, and
- a long-press state, wherein the long-press state is defined as an interaction with the tactile control for more than 250 ms.

In some embodiments, the first and/or second type of peripheral communication device is a mobile communication device

In some embodiments, the first and/or second type of peripheral communication device is one or more of a chest mounted tactical EUD, a Military LMR, a vehicle mounted intercom system, and a vest worn PTT control unit.

In some embodiments, the communication module comprises one or more processors, such as a DSP and/or an MCU.

In some embodiments, the communication system further comprises the first type of peripheral communication device connected to the communication module, or wherein the communication system further comprises the second type of peripheral communication device connected to the communication module.

According to a third, fourth, and fifth aspect, the present disclosure discloses the following.

The present disclosure provides embodiments of a hearing protection and communication system with an electronic receptacle interface to be used in a harsh and demanding environment as disclosed herein according to independent claims with advantageous embodiments as defined by the dependent claims and disclosed herein.

According to a third aspect, the present disclosure provides: a communication system for communicating in a harsh environment, comprising: a hearing protection headset to be worn by a user in the harsh environment, said headset comprising: a left and a right earcup configured to attenuate external sounds from the harsh environment.

The communication system further comprises an electronic receptacle interface configured to connect one of a first type of peripheral communication device and a second type of peripheral communication device via a cable and a connector having a plurality of connector pins, and exchange electronic signals via the plurality of connector pins. Preferrable, wherein the electronic signals comprise at lease audio data.

It is advantageous to have a hearing protection headset in combination with a communication system comprising a receptacle interface adapted for connecting a first type of peripheral communication device, such as a legacy analog military radio and a second type of peripheral communication device, such as a modern digital communication device, such as a smart phone. For personnel operating in harsh and demanding environments it is desirable to have both hearing protection and a communication system which is able to connect multiple different types of peripheral communication devices via a cable and a connector directly. A wired connection is perceived to be more reliable and more secure compared to wireless connections, which is highly preferrable when used by soldiers in harsh and demanding environments to provide clear and undistorted communication.

In a preferred embodiment of the third aspect, the communication system further comprises a communication module in contact with the electronic receptacle interface, wherein the communication module is configured to switch between exchanging the electronic signals, via the plurality of connector pins, according to a first data exchange mode and a second data exchange mode. wherein; the first data exchange mode is applied when the receptable interface is connected to a first type of peripheral communication device, and the second data exchange mode is applied when the receptacle interface is connected to a second type of peripheral communication device, and wherein the communication module is further configured to enable audio data to be received and played through a speaker in the left earcup and/or a speaker in the right earcup.

By switch between exchanging the electronic signals, via the plurality of connector pins, according to a first data exchange mode and a second data exchange mode, it may be possible to communication with both the first type of peripheral communication device and the second type of peripheral communication device, particularly in a situation where the first type of peripheral communication device is different from the second type of peripheral communication device via the same receptacle interface. For example, where the first type of peripheral communication device is configured to communicate according to a first data exchange mode using a first type of signals in the form of an analog signals, and where the second type of peripheral communication device is configured to communicate according to a second data exchange mode using digital signals. This may be particularly advantageous for personal operating in harsh and demanding environments as peripheral communication devices utilized by military and public safety professionals across different agencies, units and teams may spans widely from a first type such as older analog RF Land Mobile Radios (LMR) to a second type such as a newer modern digital radio. By having a communication system comprising a communication module in contact with the receptacle interface, enables the user to plug in any type of peripheral communication device into the same receptacle interface and exchange data according to a first- or second mode, such that at least audio data received via the peripheral communication device may be received and played to the user via the hearing protection headset. Hence, the communication system according to the third aspect provides a modern hearing protecting and communication system capable of interfacing and communicating, via a wired connection to one receptacle interface, with both legacy analog communication devices as well as newer digital communication devices all being part of the available equipment in operation across military and public safety units around the world for establishing clear and undistorted communication in harsh and demanding environments. Situations may for example arise in a harsh environment, where for users are required to swap between a first- and second type of peripheral communication device during a mission or between tasks or assignments, making the communication system according to third aspect highly desirable as being able to transmit or receive the correct voice communication in a complex communication setup, independent of communication device types and under stressful circumstances, can make the difference between life and death.

In a preferred embodiment related to the third aspect, the communication module and the receptacle interface are integrated into a single communication device located in the hearing protection headset or located in a head-worn device comprising the hearing protection headset.

This is advantageous that the hearing protection headset contains both the receptacle interface and the communication module, thereby enabling the headset to become a compact standalone device capable of interfacing and connecting different types of peripheral communication devices directly via the receptacle interface on the headset. This is particularly advantageous for operating in harsh and demanding environments, as it reduces the required number of devices to be worn by the user. Accordingly, it may free up more space for the user to carry other essential equipment such as magazines for ammunition, food and first aid supply, power supplies etc. required by modern military or public safety personal.

According to a fourth aspect, the present disclosure provides: a communication device for a communication system for communicating in a harsh environment, the communication device comprising: an electronic receptacle interface configured to: connect one of a first type of peripheral communication device and a second type of peripheral communication device via a cable and a connector having a plurality of connector pins, and exchange electronic signals via the plurality of connector pins, wherein the electronic signals comprise at lease audio data.

In a preferred embodiment of the fourth aspect, the communication device may further comprise a communication module in contact with the electronic receptacle interface , wherein the communication module is configured to switch between exchanging the electronic signals according to a first data exchange mode and a second data exchange mode, wherein; the first data exchange mode is applied when the electronic receptacle interface is connected to a first type of peripheral communication device, and the second data exchange mode is applied when the electronic receptacle interface is connected to a second type of peripheral communication device, and wherein the communication module is further configured to enable audio data to be received and played through a speaker in a headset.

In a preferred embodiment of the third aspect, the communication module and the electronic receptacle interface are integrated into a single communication device that is located separately from the hearing protection headset.

In a preferred embodiment the fourth aspect is part of the third aspect.

The fourth aspect of the present disclosure provides similar advantages and benefits as described in relation to the third aspect and the aforementioned embodiments thereof. with the additional advantages that the communication device according to the fourth aspect may be located elseward on the soldier, such as on a west worn device for providing additional communication capabilities in relation to the third aspect and related embodiments. The communication device according to the fourth aspect may be embodied in a west worn PTT control unit (e.g. A single communication device) comprising one or more user interfaces, thereby expanding the number of peripheral communication devices to be connected to the communication system while maintaining the agnostic nature of the receptacle interface.

According to a fifth aspect, the present disclosure provides: a hearing protection headset for communicating in a harsh environment and to be worn by a user in the harsh environment, said headset comprising: a left and a right earcup configured to attenuate external sounds from the harsh environment; an electronic receptacle interface configured to: connect one of a first type of peripheral communication device and a second type of peripheral communication device via a cable and a connector having a plurality of connector pins, and exchange electronic signals via the plurality of connector pins (404a-404g), wherein the electronic signals comprise at lease audio data.

In a preferred embodiment of the fourth aspect, the hearing protection headset may further comprise a communication module in contact with the electronic receptacle interface, wherein the communication module is configured to switch between exchanging the electronic signals according to a first data exchange mode and a second data exchange mode, wherein; the first data exchange mode is applied when the electronic receptacle interface is connected to a first type of peripheral communication device, and the second data exchange mode is applied when the electronic receptacle interface is connected to a second type of peripheral communication device, and
wherein the communication module is further configured to enable audio data to be received and played through a speaker in the left earcup and/or a speaker in the right earcup.

The hearing protection headset according to the fifth aspect provides a compact standalone hearing protection and communication headset to be used in harsh and demanding environment having an automatic adaptable interface for connecting any type of peripheral communication device. Such a compact hearing protecting and communication headset is highly advantageous as a soldier or police officer would only need to carry a single headset having both hearing protection and communication capabilities. The receptacle interface may automatically adapt the way the headset interface, communication and exchange electronic signals with the connected peripheral communication device independent of its type. This is beneficial, as the user does not need to worry about compatibility issues between the headset and available radios, as the headset would automatically adapt its communication scheme to any type of peripheral communication device.

In a preferred embodiment the fifth aspect is part of the third aspect.

The fifth aspect of the present disclosure provides similar advantages and benefits as described in relation to the third and fourth aspect and the aforementioned embodiments thereof.

In a preferred embodiment of the third aspect, the communication module is configured to switch between exchanging the electronic signals according to a first data exchange mode and a second data exchange mode, such that: the first data exchange mode is applied when the first type of peripheral communication device is connected to the electronic receptacle interface and the communication module receives first coupling data associated with the first type of peripheral communication device, and/or the second data exchange mode is applied when the second type of peripheral communication device is connected to the electronic receptacle interface and the communication module receives second coupling data associated with the second type of peripheral communication device.

In a preferred embodiment of the third aspect, the communication module is further configured to receive the first coupling data and/or second coupling data from the cable being connected to the electronic receptacle interface. Preferably where the electronic receptacle interface is configured to recognize the type of device being connected to the electronic receptacle interface and provide or derive the first coupling data or the second coupling data, respectively, in response thereto. More preferably where the cable comprises or is connected to a microchip comprising the first coupling data or the second coupling data.

In a preferred embodiment of the third aspect, the plurality of connector pins comprise at least comprises: a sense connector pin for obtaining the first or second coupling data associated with said peripheral communication device, and a first data connector pin, and a second data connector pin.

It is advantageous that the receptacle interface is configured to obtain coupling data via a dedicated connector pin in the connector, as the communication system may be able to switch between the first data exchange mode and the second data exchange mode in response to obtaining first coupling data or second coupling data. Thus, the communication system may adjust the way data signals are exchanges with the connected peripheral communication device in accordance with the device type based on coupling data obtained directly via a sense connector pin, such that the communication scheme may be initialized immediately upon connection between the connector and the receptacle interface. This enables the communication system to boot and adjust the correct circuits, processors and drivers to facilitate the correct communication via the first and second connector pin.

In a preferred embodiment of the third aspect, the communication system is further configured to switch between exchanging the electronic signals, such that; the first and second data connector pins are configured solely to receive audio data when the communication system is operating in the first data exchange mode, and; the first and second data connector pins are configured for exchanging both audio and non-audio data when the communication system is operating in the second data exchange mode.

In a preferred embodiment of the third aspect, the audio data comprises; analog audio signals when the communication module is exchanging the electronic signals according to the first data exchange mode, and digital audio data packages, preferably according to USB protocol, a UART protocol, or an EtherNet/IP protocol, when the communication module 106 is exchanging the electronic signals according to the second data exchange mode.

In a preferred embodiment of the third aspect, the non-audio data comprise digital non-audio data packages, preferably according to USB protocol, a UART protocol, or an EtherNet/IP protocol, such as video data, control-instructions, commands, metadata etc.

This way, the communication system is capable of adjusting the data exchange mode, such that when a first type of peripheral communication device, such as an analog radio, is connected and the first and second data connector pins are used to receive audio signals from the first type of peripheral communication device to be played to the user via the speakers in the headset. Additionally, the communication system is capable of changing the data exchange mode when connected to a second type of peripheral communication device, such as a digital communication device, such that the first and second data connector pins instead are used to exchange both audio and non-audio data. This is highly advantageous as the receptacle interface thereby configured to acts as a analog interface for receiving stereo audio signals when connected to an analog peripheral communication device or support digital communication when connected to a digital peripheral communication device, such that both audio data and other types of data like video and location data may be exchanged between the communication system and the second type of peripheral communication device. By utilizing the same pair of pins to support different communication schemes or data exchange modes reduces the overall number of pins required in the connector, as the pins are used for data exchange in a smart manner.

In a preferred embodiment of the third aspect, the communication module is further comprising an audio CODEC device configured for receiving audio data when exchanging the electronic signals according to the first data exchange mode.

In a preferred embodiment of the third aspect, the communication module is further comprising a USB controller configured for exchanging audio data and non-audio data when exchanging the electronic signals according to the second data exchange mode.

In a preferred embodiment of the third aspect, the communication module is further comprising a multiplexer unit configured to switch between exchanging the electronic signals according to the first data exchange mode and the second data exchange mode by altering a signal exchange path between the pair of the first and the second connector pins and the communication module.

It is advantageous that the communication system comprise a multiplexing (MUX) circuitry for altering the electronic signals between a peripheral communication device a CODEC device when receiving analog audio from a first type of peripheral communication device via the first and second data connector pins or a USB controller when exchanging audio data and non-audio data with a second type of peripheral communication device via the same first- and second connector pins. This way, the communication system may be compact and utilize less physical electrical terminals for supporting different types of communication.

In a preferred embodiment of the third aspect, the plurality of connector pins at least further comprises: a power connector pin for directing power to or from the first or second type of connected peripheral communication device, and an audio transmission connector pin adapted for transmitting voice data, when the communication module is exchanging the electronic signals according to the first data exchange mode, and a PTT connector pin adapted for transmitting a Push-to-Talk (PTT) signal to the first type of peripheral communication device, when the communication module is exchanging electronic signals according to the first data exchange mode, and a ground connector pin for connecting to electronic ground.

In a preferred embodiment of the third aspect, the plurality of connector pins at most comprises 7 pins.

By limiting the supported number of individual connector pins to a total number of 7 provide the advantage that the overall formfactor of the connector and receptacle interface may be relatively small and compact, such that it's cheaper to produce and may fit better into a hearing protection headset 104 for example as the size is smaller. Additionally, 7 individual connector pins provide the necessary connections (wires) to support both analog type peripheral communication devices and digital type peripheral communication devices. Also, by having a total number of 7 pins in the connector require the cable only to contain or accommodate a low number an individual wires (i.e. 7 in total), making the cable relatively thin and flexible. This is highly preferable for a user operating in harsh and demanding environments as cables are required to be mounted and routed on the west and equipment of a soldier. A relatively thin and flexible cable provides the advantages that it's easier to handle, mount and route on the vest or equipment thereby not obstructing movement or the reach to other necessary equipment.

The third, fourth, and fifth aspects (and embodiments thereof) may, respectively, be used or applied in connection with one or more of the first, the second, and the sixth aspect (and respective embodiments) including any applicable combination.

According to a sixth aspect, the present disclosure discloses the following.

The present disclosure provides embodiments of a hearing protection and communication system with earcup interfaces to be used in a harsh and demanding environment as disclosed herein according to independent claims with advantageous embodiments as defined by the dependent claims and disclosed herein.

According to a sixth aspect, the present disclosure provides: a communication system for communicating in a harsh environment, comprising: a hearing protection headset to be worn by a user in the harsh environment, said headset comprising: a left and a right earcup configured to attenuate external sounds from the harsh environment.

The communication system further comprises a left connector interface comprised by the left earcup and a right connector interface comprised by the right earcup, wherein the left and right connector interface each is configured to releasably connect with one of a first type of accessory device and/or a second type of accessory device.

This way, the user wearing the hearing protection headset may mount a first type of accessory device, such as a boom microphone to eighter the left earcup and or the right earcup. This is advantageous as the boom microphone may thereby be mounted depending on user's 100 preference. It may be advantageous to mount the boom microphone opposite of the user's 100 preferred side to handle a weapon, as the boom microphone otherwise may conflict with aiming and shooting movements requiring the gunstock to rest against the user's shoulder (e.g. on the preferred side / trigger finger side). Thereby allowing ambidextrous placement of the boom microphone. The ability to connect to a second type of accessory device, such as a 3-position switch (e.g. Toggle switch) to the left and or right earcup, allowing the user to mount a user interface directly on one of the left and/or right connector interface, thereby expanding the ability of the hearing protection headset. Other types of accessory devices may be connected, such as a light source. It is advantage for the user operation in harsh and demanding environments to be able to customize and adapt the functionality of the hearing protection headset during a missing in case a unexpected situation may arise which calls for additional or modified functionality of the communication system.

In a preferred embodiment according to the sixth aspect, the left and right connector interface individually is further configured to releasably connect a first type of accessory device and/or a second type of accessory device via a plurality of signal pins, and receive and transmit electronic signals via the plurality of signal pins. Preferably wherein the electronic signals are in the form of data signals, more preferably in the form of audio data, such as voice data, or preferably wherein the electronic signals are in the form of non-audio data,
this way the left and/or right connector interface is capable of physically connect to a plurality of signal pins which may advantageously be arranged in a configuration allowing for an unambiguous mounting directing, such that wrong mounting of said accessory devices may be avoided my choice of design. The plurality of signal pins may additionally provide a way to communicate with said accessory devices, by sending and revising audio data, such as voice data from then user when a first type of accessory device like a microphone is mounted or by sending and revising non-audio data when second types of accessory device, such as a toggle switch or lights source is mounted. It is advantageous that both the left and right connector interface is adapted to communication either audio data or non-audio data, at this provides a great flexibility for the user to mount and use audio type accessories and/or non-audio type accessories to each individual interface, thereby expanding the functionality of the communication system.

In a preferred embodiment of the sixth aspect, the communication system further comprises a communication module in contact with the left and the right connector interface, wherein the communication module is configured to automatically apply and switch between a first communication mode and a second communication mode. The first communication mode is applied when the left and/or the right connector interface(s) is/are connected to the first type of accessory device, and when in the first communication mode, the communication module enables at least a subset of the plurality of pins to receive and/or transmit one type of electronic signals, such as audio data or voice data. The second communication mode is applied when the left and/or the right connector interface(s) is/are connected to the second type of accessory device, and, when in the second communication mode, the communication module enables at least the subset of the plurality of pins to receive and/or transmit another type of electronic signals, such as non-audio data,

By being configured to automatically apply and switch between a first communication mode and a second communication mode, the communication module may automatically adapt the way the communication system is operating in response to a first and/or a second accessory device type is mounted to either the left and/or right connector interface, such that the user simply has to mount a first or second type of accessory device into either of the left and/or right connector interface, and the communication module automatically adjust the communication scheme (e.g. Audio / non-audio) accordantly enabling instantaneous functionality and operation of the mounted accessory device without any manual configuration of adjustment. The user may even replace a first type of accessory device with a second type of accessory device and the communication module will automatically adjust how to communicate, such that the left and/or right connector interface may appear accessory device type agnostic, providing ease of use and flexibility for the user wearing the communication system.

In a preferred embodiment the communication module is further configured to connect with at least a peripheral communication device and enable audio data to be received from the peripheral communication device and played through a speaker in the left earcup and/or a speaker in the right earcup.

This may be particularly advantageous for personal operating in harsh and demanding environments as peripheral communication devices are utilized by military and public safety professionals across different agencies, units and teams such as older analog RF Land Mobile Radios (LMR), Mobile Ad Hoc Networking (MANET) radios, tactical smartphone (EUD) or a multiple-input and multiple-output (MIMO) radios etc. Such peripheral communication devices are used for establishing clear and undistorted communication among team members, control centers and other units operating in harsh and demanding environments as the ability to transmit or receive the correct voice communication, such as orders or instructions, even under stressful circumstances, can make the difference between life and death.

In one embodiment the communication module is further configured to transmit one or more control-instruction(s) to the peripheral communication device.

It is particularly advantageous that the communication module may be configured to enable the transmit control-instructions to a peripheral communication device, as the user may remotely (e.g. via the user interface) control the connected peripheral communication device. Thus, if a user interface type accessory device, like a toggle switch, is mounted to the left or right connector, the user may control a connected radio via the toggle switch. Personal operation in a harsh environment may typically carry a load of personal equipment such as helmet, uniform, boots, armor, weapon(s), ammunition, food, canteens, compass, first aid kit and communication devices, etc. placed strategically on the uniform such that the different effects are easy to reach. The front part of a soldier is typically a crowded space, and the most critical equipment is prioritized, such as ammunition. Communication devices, such as radios, which may vary a lot in weight and form factor, are typically located out of immediate reach for the soldier, such as on the back making remote operation (e.g. Via a second type of accessory) of the communication device highly desirable.

In one embodiment, the communication module is integrated into a single communication device located in the hearing protection headset 104 or located in a head-worn device comprising the hearing protection headset 104.

In this embodiment, the hearing protection headset contains the communication module, enabling the headset to become a compact standalone unit capable of directly interfacing and communicating with different types of accessory devices and at least a peripheral communication devices directly. This is particularly advantageous for operating in harsh and demanding environments, as it reduce the required number of devices to be worn by the user, Accordingly, it may free up more space for the user to carry other essential equipment such as, magazines for ammunition, food and first aid supply, power supplies etc. required by modern military or public safety personal

In one embodiment, the communication module is configured to automatically apply and switch between the first communication mode and the second communication mode when the communication module receives first identification data associated with the first type of accessory device, and when the communication module receives second identification data associated with the second type of accessory device, respectively.

This is advantageous as, by obtaining first identification data and second identification data, the communication module may be able to automatically apply and switch between the first communication mode and a second communication mode in response to the obtained identification data. Such that first identification data associated with the first type of accessory device may be enable to communication module to automatically apply the first communication mode and second identification data associated with the second type of accessory device may enable to communication module to automatically apply the second communication mode.

In one embodiment, the communication module is further configured to receive the first identification data and/or second identification data from said accessory device connected to the left and/or right connector interface, preferably where both the left and/or right connector interface is configured to recognize the type of accessory device respectively being connected to the left and/or right connector interface, more preferably where said accessory device comprises a electronic circuitry comprising the first identification data or the second identification data.

This way, the communication module may be able to obtain first identification data and second identification data when eighter or both of the left and right connector interface recognizes a connected accessory device. Thus, the left and right connector interface may recognize if a first second type of accessory device is connected and/or a second type of accessory device is connected and thereby enabling the communication module to automatically apply and switch between the first communication mode and the second communication mode. This is advantageous, as the communication module may recognize a first type of accessory device, such as a microphone unit is connected to the left and/or right connector interface and switch the communication mode accordingly, such that the microphone may be used to record the voice signal of the user when speaking. Similarly, the communication module may recognize a second type of accessory device, such as a toggle switch is connected to the left and/or right connector interface and switch the communication mode accordingly, such that the communication module may send control-instructions to a connected peripheral communication device, like a Push-to-Talk (PTT) signal such that voice data from the user may be send wirelessly via a radio using radio-waves.

In one embodiment, the plurality of signal pins for the left and right connector interface , each at least comprises; a sense signal pin for obtaining identification data associated with said accessory device, and a first signal pin, and a second signal pin.

In one embodiment, when in the first communication mode, at least the subset of the plurality of pins is the first and second signal pin, and wherein the one type of electronic signals comprise voice data, and wherein when in the second communication mode, at least the subset of the plurality of pins is the first and second signal pin, and wherein the other type of electronic signals comprise non-audio data.

In some embodiments, the sense signal pin is a TS (Tip-Sleeve) jack type pin, allowing two separate electrical to be contained within the formfactor of a single physical pin. This is advantageous at may reduce the total number of physical connector pins to 3, thereby allowing the physical embodiment of the plug-end of a accessory device in an asymmetric manner such that the user may only fit the accessory device into the left and/or right connector interface in one (correct) orientation such that wrong mounting may be avoided by chose of design.

In one embodiment, the first type of accessory device is a microphone device for obtaining voice data, preferably from the user when speaking, preferably wherein the microphone is attached to a flexible rod configured to be positioned close to the mouth of the user.

In one embodiment, the second type of accessory device is a user interface, light source, a camera, a video camera or a data sensor.

In one embodiment, the user interface is configured to exchange non-audio data with the communication module, such that the user interface allows the communication module to transmit the one or more control-instruction(s) to at least one peripheral communication device.

In this way, a user may physically interact with the communication system to provide user input to the communication module, which is advantageous as the user wearing the communication system in a harsh environment, may be enabled to provide instructions to the communication module via the user interface

In one embodiment, the user interface comprises one or more tangible controls that are manually operably by the user.

It may be particular advantageous to have tangible controls on the user interface when used by personal in harsh environments, as the tangible controls should be manually operable by the user under difficult and stressful circumstances to enable clear and undistorted communications, even while wearing thick gloves or being in darkness such that the structure and design of the controls are tangible to enable unambiguously manual operation.

In one embodiment, one of said tangible controls is a switch, wherein the switch allows the communication module to transmit the one or more control-instructions in the form of Push-to-Talk (PTT) signal to the at least the peripheral communication device.

In one embodiment, the communication module is integrated into a single communication device that is located separately from the hearing protection headset.

The sixth aspect (and embodiments thereof) may be used or applied in connection with one or more of the first, the second, the third, the fourth, and the fifth aspect (and respective embodiments) including any applicable combination.

### BRIEF DESCRIPTION OF THE DRAWINGS

Embodiments of the disclosure will now be described in more detail. Various embodiments of the systems and/or the methods according to the different aspects as disclosed herein will be described in connection with the appended drawings, in which:
FIG. 1 schematically illustrates an example of a user 100 wearing a communication system 102 configured to be worn by a person operating in a harsh and demanding environment and in communication with other connected units via a peripheral communication device 110**.**
FIG. 2A schematically illustrates an example of a communication system 102 to be worn by a person 100 operating in a harsh and demanding environment, here as an example embodied in a hearing protection headset 104.
FIG. 2B schematically illustrates an exploded view of the left earcup 200a of a communication system 102 embodied as a hearing protection headset 104, showing the internal components.
FIG. 2C schematically illustrates an exploded view of the right earcup 200b of a communication system 102 embodied as a hearing protection headset 104, showing the internal components.
FIG. 3A schematically illustrates an exemplary functional block diagram of the communication module 106 of the communication system 102 according to the present disclosure.
FIG. 3B schematically illustrates an example of a system diagram of the communication module 106 forming part of the communication system 102 according to the present disclosure.
FIG. 4A schematically illustrates an example of the electronic receptacle interface 218 of the communication system 102 according to the present disclosure.
FIG. 4B schematically illustrates an example of a connector 220 having a plurality of connector pins 404a-404g and configured to connect a peripheral communication device 110 to the communication system 102 according to the disclosure, e.g. or preferably by connecting to the receptacle interface 218 of FIG. 4A.
FIG. 4C schematically illustrates an example of a communication module 106 comprising a multiplexing (MUX) circuitry 406 and in connection with an electronic receptacle interface 218 according to an embodiment of the disclosure.
FIG. 5A Schematically illustrates an example of a communication module 106 and a user interface 108 comprising tangible controls (224a-224c) according to the present disclosure.
FIG. 5B schematically illustrates an example of the mapping between the tangible control elements (e.g. 224a-224c), specific operation states (e.g. 508a-508c) and associated control-instructions 514 for a first type of peripheral communication device and a second type of peripheral communication device peripheral communication device of the communication systems 102 according to the present disclosure.
FIG. 6A schematically illustrates an example of communication system 102 comprising a left connector interface 216a and a right connector interface 216b in connection with a communication module 106 according to the present disclosure.
FIG. 6B schematically illustrates an example of a left earcup 200a of a hearing protection headset 104 comprising a left connector interface 216a and a first type of accessory device to the present disclosure.
FIG. 6C schematically illustrates an example of the communication module 106 configured to automatically apply and switch between exchanging the electronic signals with the left connector interface 216a according to a first communication mode and a second communication mode respectively.
FIG. 7A schematically illustrates a first configuration 702 of a communication system 102 according to the present disclosure, here as an example embodied in a circumaural hearing protection headset 104 to be worn by a user 100.
FIG. 7B schematically illustrates a first second configuration 704 of a communication system 102 according to the present disclosure.
FIG. 7C schematically illustrates a first second third configuration 706 of a communication system 102 according to the present disclosure.

### DETAILED DESCRIPTION

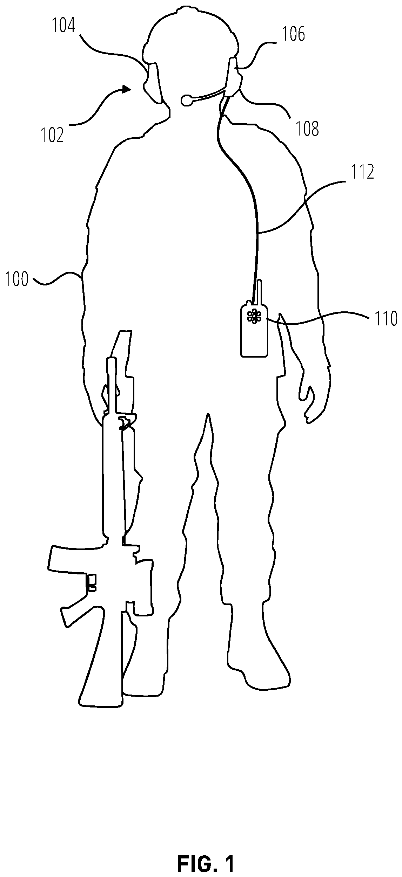

Various aspects and embodiments of a communication system as disclosed herein will now be described with reference to the figures FIG. 1-FIG. 7C.

In one embodiment, the communication system is configured to be worn by a person operating in a harsh and demanding environment, in communication with other users connected via peripheral communication devices. In a two-way radio communication setup, two or more individuals or users are equipped with handheld transceivers (e.g. Peripheral communication devices), such as radios. Each peripheral communication device may operate on designated frequencies or talk group, enabling the transmission and reception of voice messages over radio waves (i.e. Radio frequency). When one user activates their radio, their voice may be converted into electrical signals, which are then modulated into radio waves and transmitted via the peripheral communication device. A second peripheral communication device, tuned to the same frequency or talk group, may receive these waves, demodulate them, and converts them back into audible sound. This radio frequency (RF) communication link may allow for clear and undistorted communication between users over varying distances. Hence, in one embodiment, the peripheral communication device is configured to establish person to person voice communication, audio and data communication links with other peripheral communication devices.

In one embodiment a user may wear a wearable communication system comprising a circumaural hearing protection headset configured to attenuate external sounds from the harsh environment, such as loud pulse noises from gunfire, explosions or loud vehicle noises, thereby protecting the hearing of the user 100.

In one embodiment the communication system 102 may comprise a hearing protection headset, in the form of a pair of earcups adapted to be situated over the ears of the user, a communication module, configured to establish and maintain one or more communication links via a connected peripheral communication device 110 and a user interface 108 for operating and controlling the connected peripheral communication device 110. This advantageously enables the user to be in a so-called "dismounted" configuration that refers to a situation where the user may maintain a communication link (e.g. person to person voice communication, audio and/or data communication links) with one or more remotely located other users via their respective communication systems and peripheral communication devices without having physical link, such as a cable, etc. Hence, being "dismounted" enables the user to freely move around while being able to maintain a communication link.

FIG. 1 schematically illustrates an example of a user 100, being a soldier, wearing a communication system 102 configured to be worn by a person operating in a harsh and demanding environment, in communication with other users (not shown) via a peripheral communication device 110. The user 100 is wearing a communication system 102 integrated into a hearing protection headset 103 and one type of radio 110 connected to the communication system 102 via a cable 112. Alternatively, the cabled connection may be replaced by suitable wireless connection. The user 100 may carry and use one or different types of peripheral communication devices 110 operably connected to the communication system 102 for establishing person to person voice communication, audio and data links with other remote users as previously described.

### Peripheral communication devices and types

In one embodiment a peripheral communication device 110 may be of a first type of peripheral communication device, a second type of peripheral communication device or a third type of peripheral communication device each requiring different kinds of control-signals and control-instructions to be remotely controlled via an externally located communication systems 102. In one embodiment a first type of peripheral communication device may require to exchange electronic signals using analog signals and receive control-instructions by modulating a voltage signal between different levels via resistor circuits (e.g. First control-signal) and a second type of peripheral communication device may require to exchanging data and control-instructions using a digital communication protocol using any of the IEEE standards, or other digital data exchange protocols requiring special data packages (e.g. Second control-signal) for sending control-instructions.

In some embodiments, the first and/or second type of peripheral communication device is a mobile communication device.

In one embodiment a peripheral communication device 110 may be a mobile communication device configured to be carried around and provide a wireless communication link (e.g. A radio frequency (RF) communication link) to another peripheral communication device. Preferably, a peripheral communication device 110 may be understood as a device for providing voice and/or data transmission wirelessly over long distances using radio frequency bands or other electromagnetic wave bands. In one embodiment, a peripheral communication device 110 may be a handheld 2-way radio for providing voice and data communication in the VHF and UHF bands using different waveforms. Thereby, a peripheral communication device 110 may offer secure and reliable communication in various harsh operational environments. Examples of peripheral communication devices 110 may be an L3Harris^{®} Falcon III AN/PRC-152A that provides simultaneous voice, video and high-speed data in a highly portable form factor for dismounted use which provides interoperable communication in multiple frequency bands and supports voice and data transmission. Other examples of a peripheral communication device 110 may be a specialized radio system to be used in harsh and demanding environments by first responders or military personnel, such as the analog Motorola GP340 or the digital DP4000E portable two-way radios, the TrellisWare Shadow 950/900 tactical radio, or the Barrett PRC-2090 HF tactical radio, each offering long-range communication capability in the HF band for dismounted operations. Yet other types of peripheral communication devices 110 may be a chest mounted Tactical Display Unit (TDU) or End-User-Device (EUD), such as a Samsung Galaxy S23 Tactical Edition or Samsung XCover6 Pro Tactical Edition smart phones with a 6.1-inch touch screen or alike, supporting conventional cellular capabilities as well as secure tactical communication, software applications etc. It may be advantageous for the person operating in a harsh and demanding environment to carry a a chest mounted Tactical Display Unit (TDU) or End-User-Device (EUD) for providing visual information to the user via applications like the Android Team Awareness Kit (ATAK) and the Battlefield Assisted Trauma Distributed Observation Kit (BATDOK) which advantageously enhances real-time situational awareness for the user through collection and display of information using functions like as mapping, messaging, and geofencing. This enables all team members (e.g. Multiple users) in a group to individually access relevant information simultaneously on a single screen. Thus, the different types of peripheral communication devices 110 provide reliable communication links between a user, team members, units and central command centers to facilitate coordination and situational awareness on the battlefield or in emergency situations.

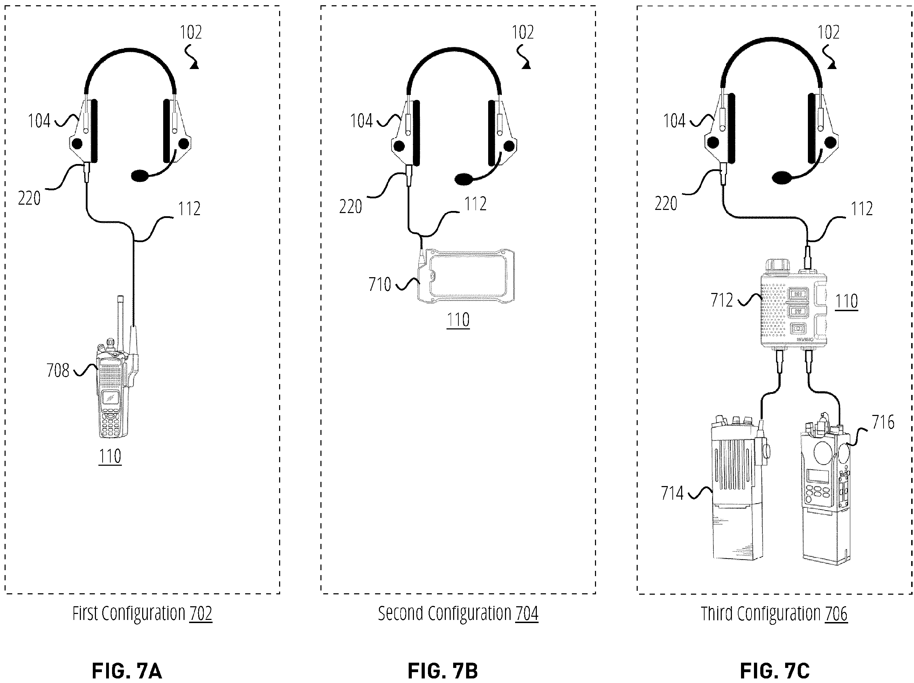

In one embodiment the first and/or the second type of peripheral communication device is one or more of a chest mounted tactical EUD (e.g. 710 See FIG. 7B), a Military LMR (e.g. 708 See FIG. 7A), a vehicle mounted intercom system, and a vest worn PTT control unit (e.g. 712 See FIG. 7C).

### Hearing protection and communication headset

In a preferred embodiment, the communication system 102 for communicating in a harsh environment, comprise a hearing protection headset 104 to be worn by a user 100 in the harsh environment, said headset 104 comprising: a left 200a and a right earcup 200b configured to attenuate external sounds from the harsh environment.

In another embodiment, the communication system 102 comprise a communication module 106 configured to connect with a peripheral communication device 110 and receive at least one audio signal from the peripheral communication device.

In some embodiments, the communication module 106 may be configured to transmit the at least one audio signal to a speaker in the left earcup 200a and/or a speaker in the right earcup 200b.

In one embodiment, the communication system 102 is integrated into a single hearing protection headset 104.

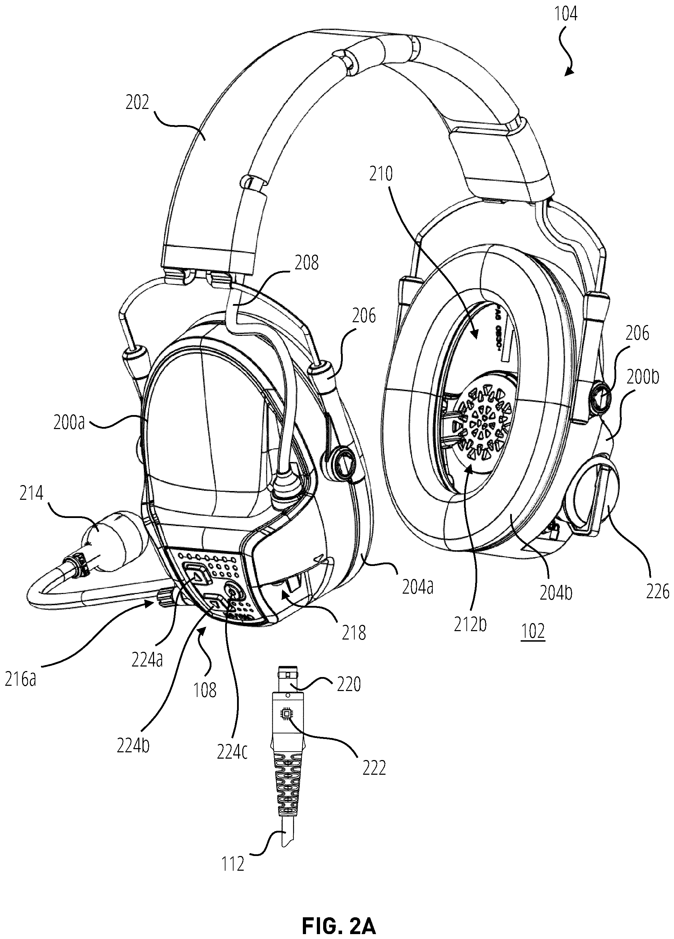

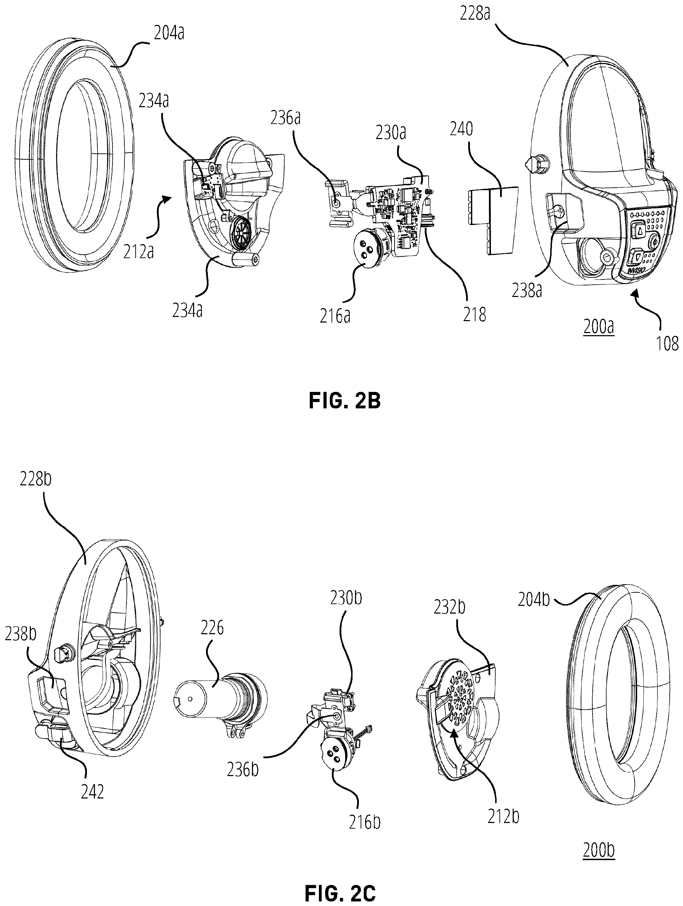

FIG. 2A-FIG. 2C schematically illustrates an example of a communication system 102 to be worn by a person 100 operating in a harsh and demanding environment, embodied in a hearing protection headset 104. FIG. 2B show examples of the individual components comprised in the left earcup 200a and FIG. 2C show examples of the individual components comprised in the right earcup 200b in a sequential special arrangement to highlight some internal components and their structural and functional relationship

In one example illustrated in FIG. 2A, the hearing protection headset 104 may include a left earcup 200a and a right earcup 200b and a headband 202. The headband 202 may couple the left earcup 200a with the right earcup 200b both physically, via a coupling arrangement 206, and electronically via a cup-to-cup cable 208. The headband 202 can be arced, such as to extend over the top of a users 100 head while the headset is in use (e.g. worn by the user 100). The headband 202 may be flexible, such as to allow the user 100 to spread the left earcup 200a from the right earcup 200b when the user 100 is putting on the headset 104. The headband 202 may include padding, such as to at least partially conform to the user's head and increase the user's comfort. Both the left earcup 200a and right earcup 200b may be configured to fit around a user's 100 ear and be disposed on the side of a user's head while in use. Both the left earcup 200a and the right earcup 200b may each respectively define a cavity 210 for the user's 100 ears when wearing the hearing protection headset 104. Both earcups 200a, 200b may include a sealing ring (e.g. A left cushion 204a and a right cushion 204b) adapted to extend around the user's 100 ear. The cushions 204a, 204b may be flexible and able to conform to the user's 100 head and provide an acoustic seal between the earcups 200a, 200b and the user' s 100 head, such as to attenuate the amount of noise or sound waves that reach the user's 100 ear, to at least partially protecting the user's ear from external noises. Thereby providing passive hearing protection. Even though not explicitly seen from FIG. 2A, the hearing protection headset 104 may contain structures (e.g. See FIG. 2B and FIG. 2C), features and functionalities at described at least in part in both document US20220217459A1 and US11968493B2 (hereby incorporated by reference in its entirety). The hearing protection headset 104 may include a speaker unit, such as a left speaker unit 212a located in the cavity 210 of the left earcup 200a and a right speaker unit 212b located in the 210 of the right earcup 200b. Both speaker units 212a, 212b being directed towards the ears of the user 100 when waring the headset 104 such that a sound signal can be played via one or both of the speaker units left speaker unit 212a, 212b enabling the user 100 to receive auditive information when waring the headset 104.

In some embodiments, hearing protection headset 104 may include a transmission (Tx) microphone, such as boom microphone 214 integrated into a flexible arm or rod adapted to position the microphone relatively close to the mouth of the user 100.

In the illustrated example in FIG. 2A, the boom microphone 214 may be configured to pick up communications from a user 100, such as recording a voice signal to be transmitted using radio frequency to a remote receiver, such as via a connected peripheral communication device 110. In a preferred embodiment, the boom microphone 214 may be releasably connect to a connector interface (e.g. 216a or 216b) on the headset 104.

In various embodiments the hearing protection headset 104 may include individual connector interfaces (e.g. A left connector interface 216a and a right connector interface 216b) on each of two earcups 200a, 200b such that a boom microphone 214 may be positioned on the left earcup 200a and/or on the right earcup 200b. This is advantageous as the boom microphone 214 may thereby be mounted depending on user's 100 preference. It may be advantageous to mount the boom microphone 214 opposite of the user's 100 preferred side to handle a weapon, as the boom microphone 214 otherwise may conflict with aiming and shooting movements requiring the gunstock to rest against the user's 100 shoulder (e.g. on the preferred side / trigger finger side). Thereby allowing ambidextrous placement of the boom microphone 214.

In another embodiment, the hearing protection headset 104 may contain a receptacle interface 218 for releasably connecting a peripheral communication device 110 via a cable 112 containing a connector or plug 220. The receptacle interface 218 may allow the user 100 to connect an external peripheral communication device 110 into the communication system 102.

In one embodiment, the receptacle interface 218 may be a socket adapted to engage mechanically and electronically with the connector or plug 220.

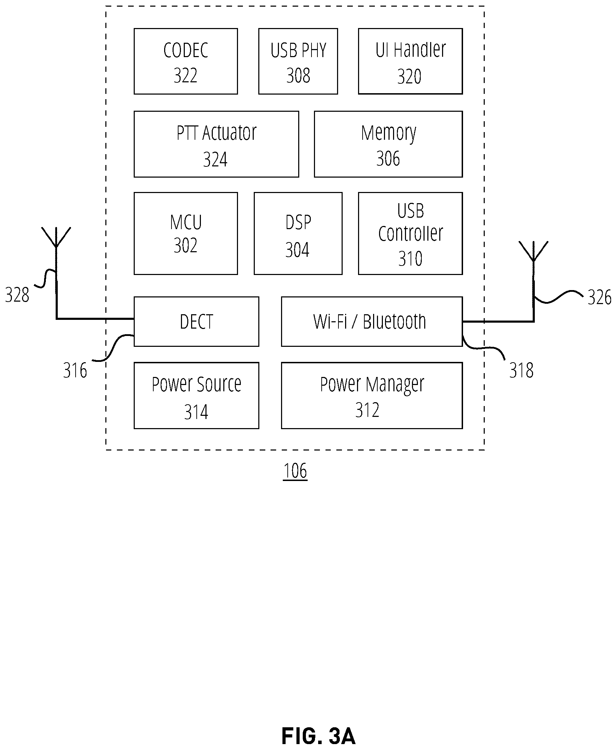

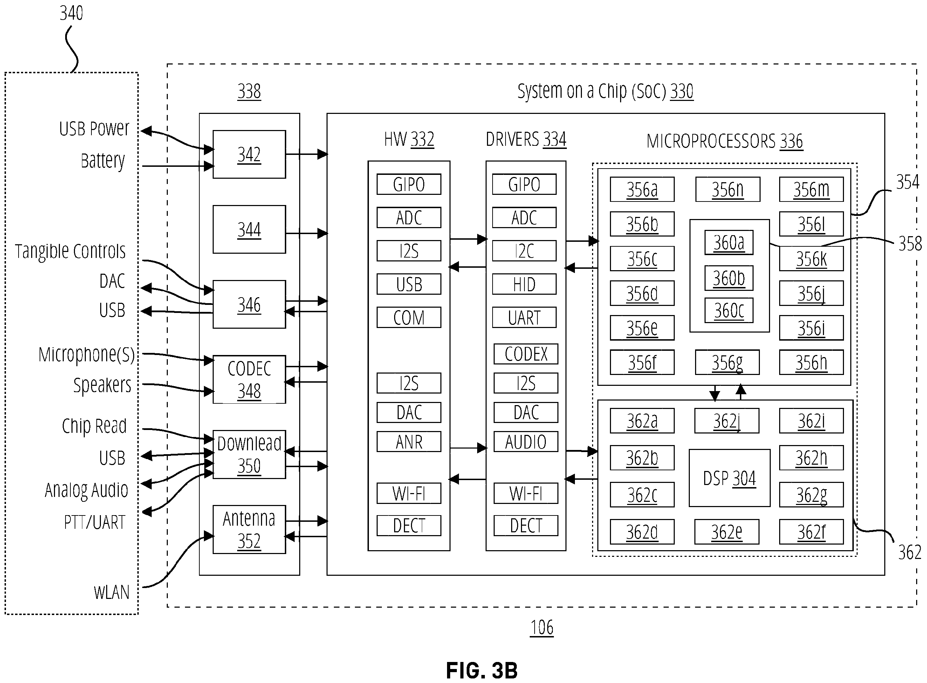

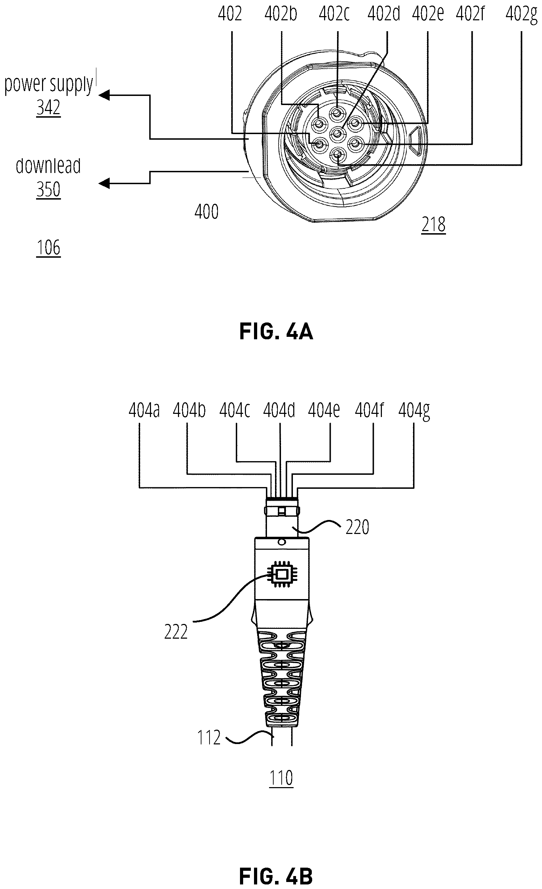

In the illustrate example in FIG. 2A the hearing protection headset 104 comprising the communication module 106 additionally comprise a receptacle interface 218, configured to releasably connect a peripheral communication device 110 such as an AM/FM radio, a two-way radio and/or a cell phone as described elsewhere via a cable 112 and a connector 220. The receptacle interface 218 may be in contact with a communication module 106 (e.g. see FIG. 3A and FIG. 3B) comprised by one or both of the earcups 200a, 200b and configured to connect and exchange control-signals, control-instructions, audio signals and other information and data with the peripheral communication device 110 via a plurality of connector pins (e.g. see FIG. 4A and FIG. 4B).

In various embodiments, the peripheral communication device 110 may be connected to the hearing protection headset 104 through a wireless connection, such as Bluetooth connection or wireless telephony standard like DECT (Digital Enhanced Cordless Telecommunications).

In some embodiments, when a peripheral communication device 110 is connected to the communication module, coupling data associated with the peripheral communication device 110 may be obtained by the communication module 106. In one embodiment, such coupling data may contain device type information specifying the type of peripheral communication device 110 (e.g. A first type of peripheral communication device or a second type of peripheral communication device). In the situation where a peripheral communication device 110 may be connected via a connector 220 configured to interface with the receptacle interface 218 on the headset 104, information and functional settings (e.g. Control-instruction(s) and communication protocol(s)) or information thereof) may be transmitted via the connection interface e.g. or preferably as described at least in part in EP2845115B1 (hereby incorporated by reference in its entirety) from a microchip (e.g. cable chip 222) embedded or located in the connector 220 (or alternative embedded or located in the cable 112 of the connector 220 or elsewhere).

In one embodiment, the cable chip 222 may be a microchip comprising an embedded memory configured for storing programmable data representing code, settings, instructions, and/or other data.

In a preferred embodiment, the communication module 106 may obtain coupling data by reading the cable chip 222 data when connected via the receptacle interface 218. As mentioned, a peripheral communication device 110 may be connected via a wireless interface, such as a antenna module of the communication module 106 in such a situation coupling data may be obtained according to a virtual version of the cable chip 222 stored in a memory associated with the peripheral communication devices 110 that may be transmitted to the communication module 106 via a secure wireless link created upon paring between the peripheral communication device 110 and the headset 104.

In some embodiment, one or both of the earcups, such as the left earcup 200a and/or the right earcup 200b may contain a user interface 108 allowing the user 100 to provide manual user inputs for controlling the connected peripheral communication device 110 and other functions of the headset 104 e.g. perform man-machine interactions (MMI). For example as shown in FIG. 2A the user interface 108 is displayed in the form of a rubber keypad, with three tangible control elements in the form of pushbuttons (224a-224c), such as an up-button 224a and down-button 224b" and a middle-button 224c. In the present example, the user interface 108 may be configured to allow the user 100 to easily interact with the buttons (224a-224c) based on touch and feel rather than visual confirmation which is advantageous as the user interface 108 may be positioned outside the users field of view, such as the side of head (e.g. on the headset 104 when worn).

In an alternative embodiment, the tangible control element may be a multi position rotatable knob, a toggle switch or mini joystick configured to support multiple operation states such as a momentary toggle switch, where the pin returns to a default position when released or may stay in one state until manually changed.

In a preferred embodiment, the hearing protection headset 104 may contain a battery compartment 226 for holding a power source such as a single use or multiuse battery for powering the communication system 102. It may be advantageous to configure the communication system 102 to use standard alkaline batteries such as an AA or AAA batteries or alike, as such batteries are easy to procure and replace around the world when depleted, thereby securing a reliable power source in demanding and harsh environment.

FIG. 2B show an example of the individual components comprised in the left earcup 200a in a sequential special arrangement to highlight some internal components and their structural and functional relationship. The left earcup 200a containing a left primary housing 228a configured to accommodate, fixate and seal the internal components. The housing 228a may be made in an acoustically attenuating material such as rugged plastic or polymer material, preferable reinforced with carbon for reducing the weight and increasing the strength and durability while having good acoustically damping properties. A user interface 108 may be located on the external surface of the housing 228a, such that it is easy to always reach for the user 100. Inside the housing 228a, a primary printed circuit board (PCB) 230a may be mounted and sealed from the external environment by the left speaker unit assembly 232a containing the left speaker unit 212a. The left cushion 204a may be adapted to engage with the left primary housing 228a via a snap lock, enabling the user 100 to easily replace the cushions (e.g. 224a and 224b) when worn down or to mound another type of cushion 123a without using any tools. As previously mentioned, the left cushion 204a may form a compatible acoustic seal between the user 100 and the left primary housing 228a, such that the cushion 204a and housing 228a together form an left earmuff for passive sound attenuation providing hearing protection on the left ear of the user 100 when worn. The left speaker unit assembly 232a may be configured to hold the left speaker unit 212a, being a transducer or loudspeaker for transforming an electronic signal to an acoustic signal such that voice and sound are transmitted electronically via the headset 104 and played to the user 100 when wearing the headset 104.

In one embodiment, the left speaker unit assembly 232a may additionally a water and dust tight seal around the internal electronic components (e.g. the primary PCB 230a) enabling the hearing protection headset 104 to have at least a IP68 rating specifying water resistant in fresh water to a maximum depth of 1.5 metres for up to 30 minutes, and are protected from dust, which is preferred when the headset 104 is adapted to be used in harsh and demanding environments.

In one embodiment the left speaker unit assembly 232a may additionally accommodate a left monitor microphone 234a and related circuitry.

In one embodiment, the left monitor microphone 234a may be a transducer for converting an acoustic response into an electric signal.

In another embodiment the left monitor microphone 234a may be positioned and exposed to the internal cavity 210 inside the left earcup 200a. Thereby configuring the left monitor microphone 234a to monitor the sound environment inside the left earcup cavity.

In another embodiment a left ambient microphone 236a may be connected to the primary PCB 230a and mounted on a rail for interlocking and sealing with the housing 228a.

In one embodiment the left ambient microphone 236a may be a transducer for converting an acoustic response into an electric signal and positioned in a forward-facing position on the left earcup 200a. Thus, the left ambient microphone 236a may monitor the external sound environment surrounding the left earcup 200a.

In the example shown in FIG. 2B the left primary housing 228a may contain a recess 238a on the external surface around the active sensing area of the left ambient microphone 236a for holding a wind filter (not shown), such as a foam or porous material for mechanically damping or removing turbulent noise from wind etc. A left connector interface 216a may additionally be connected to the main primary PCB 230a. The left connector interface 216a containing three receptor terminals for connecting to the boom microphone 214.

In a preferred embodiment, the left connector interface 216a is configured releasably connect a first type of accessory device and/or a second type of accessory device.

In one embodiment the electronic receptacle interface 218 and corresponding circuitry may additionally be connected to the primary PCB 230a. The electronic receptacle interface 218 may be configured for connecting to one or more different types of peripheral communication devices 110 and exchange electronic signals, via a plurality of connector pins.

It is advantageous that the hearing protection communication system 102 in one embodiment contains an electronic receptacle interface 218 for connecting one or more types of peripheral communication devices 110 via a cable 112 and connector 220. For example, the system 102 may be configured to be used in harsh and demanding environments, wherein it is often required that communication links between devices (e.g. Headset 104 and communication devices 110), for enabling clear and understated communication. Wired connections may lower emissions of wireless signals such that the wireless signals cannot be traced by enemy forces. Cables 112 provides a direct, secure and reliable data exchange link between devices and may provide plug and play configuration settings, such that different types of peripheral communication devices may be connected using the same type of connectors 220 and receptacle interfaces 218.

In one embodiment, the primary PCB 230a contains one or more processors, configured to execute computer readable instructions causing the different components to perform the desirable actions and functions to provide the capabilities of the communication system 102 according to the disclosure as explained in more detailed in relation to FIG. 3A, FIG. 3B and throughout the disclosure. An electromagnetic compatibility (EMC) shield 240 may be arranged to cover the primary PCB 230a configured to shield the user 100 and other internal components from potential electromagnetic radiation which may cause noisy signals.

In one embodiment, the right earcup 200b may contain similar components and related embodiments as represented in relation to the left earcup 200a (e.g. In FIG. 2B) which is performing the substantially equivalent tasks as the left counterparts, just related to the right-hand side of the user 100. An example of the right earcup 200b is shown in FIG. 3B displaying the internal components, such as the right primary housing 228b and wind filter recess 238b, right cushion 204b, right speaker unit assembly 232b, right monitor microphone 234b, right ambient microphone 236b and right connector interface 216b, such that the right cushion 204b and right primary housing 228b together form an right earmuff for passive sound attenuation providing hearing protection on the right ear of the user 100 when worn.

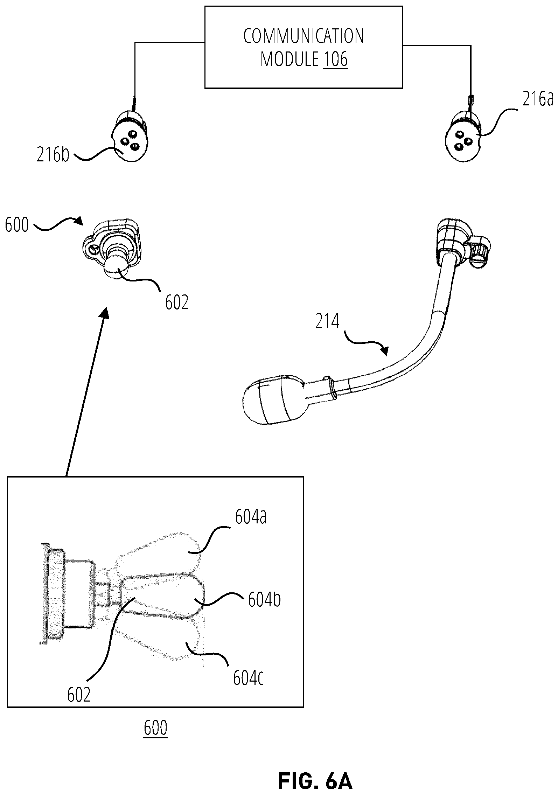

In one embodiment, The right earcup 200b may contain a battery compartment 226 configured to accommodate a battery type power source for powering at least the headset 104. The right primary housing 228b may contain a cover cap 242 for covering the left connector interface 216a not used for the boom microphone 214 (or any other type of accessory device, e.g. See FIG. 6A) such that the headset 104 maintains the IP68 rating. The right earcup 200b may additionally contain a secondary PCB 230b connected to the electronic components in the right earcup 200b and connected to the primary PCB 230a and other components in the left earcup 200a via the cup-to-cup cable 208. The secondary PCB 230b may contain one or more additional processors and circuitries operably coupled with the primary PCB 230a for enabling the components of the headset 104 (i.e. in both earcups 200a and 200b) to function together and provide the capabilities of the communication system 102.

In one embodiment, the primary 230a and/or secondary PCB 230b may thus constitute at the communication module 106 of the communication system 102, explained in more details in relation to FIG. 3A, FIG. 3B and throughout the disclosure.

### Communication module

In some embodiments, the communication module comprises one or more processors, such as a DSP and/or an MCU.

In one embodiment, the communication module 106 may contain a main control unit (MCU) 302 such as a microcontroller system (e.g. ST from STMicroelectronics) such as an ARM processor core based on a Reduced Instruction Set Computing Architecture (RISC) and/or an FPGA processer or similar, for executing instructions, controlling other units, and/or performing complex computational tasks. The MCU 302 may be the central unit configured to run embedded software/firmware and responsible for control and coordination data exchange between circuits and electronic components.

In another embodiment, the MCU 302 may be in connection with a dedicated digital signal processing (DSP) unit 304 for advanced analog and/or digital signal processing.

FIG. 3A schematically illustrates a processing architecture example of the communication module 106 of the communication system 102 according to one or more embodiments of disclosure. The DSP 304 may be a dedicated processor for handling audio mixing and processing tasks to ensure correct audio distribution across, for example speaker units (e.g. 212a, 212b) and microphones (e.g. 234a, 234b, 236a, 236b, 214) of a headset 104 (see FIG. 2A-FIG. 2C). The MCU 302 and DSP 304 units may be separate processor chips or operate in conjunction in a single chipset.

In some embodiments, the MCU 302 and/or DSP 304 may be configured to operate at least one artificial neural network. A artificial neural network may be trained and configured to execute algorithms such as one or more of speech recognition, voice-to-text, image classification, enhanced situational awareness, 3D directional sound processing, advanced signal processing, active noise cancellation, etc. In the present example in FIG. 3A, the processors (e.g. 302, 304) may be in connection with a memory 306 component for storing code, setting and instructions such as a flash memory and a random-access memory (RAM). The MCU 302 may further be in connection with additional electronic components such as a USB PHY 308 hardware component that may act as an interface between the digital signals of a USB controller 310 and signals on an USB bus used for administrating and negotiation of digital data communication protocols with connected peripheral communication devices 110 with digital capabilities. A Universal Serial Bus (USB) communication scheme is "host controlled" meaning that there can only be one USB host per bus (i.e. physical connections and communication pathways that enable data transfer between various components).

The USB controller 310 may, in one embodiment, comprise a Host Negotiation Protocol (e.g. such as the On-The-Go specification) which allows two devices (e.g. the communication module 106 and the peripheral communication device 110) to negotiate the roles of "USB host" and "USB peripheral" (i.e. counterpart of USB host). The USB host may be responsible for undertaking all transactions and scheduling bandwidth. Data can be sent by various transaction methods using a token-based protocol as generally known in the art. In an advantageous embodiment, the communication system 102 may be configured as a USB peripheral, preferably a USB audio peripheral (e.g. a USB headset) when connected to a peripheral communication device 110 acting as a USB host, such as a smartphone (e.g. EUD 710 in fig. FIG. 7B), Tablet or personal computer, as the communication module 106 may utilize the set of standard drivers and software components typically comprised on a smartphone (e.g. EUD 710), Tablet or personal computer thereby not requiring any pre-configuration of a USB host type peripheral communication device 110 to enable data exchange.

In some embodiments, the communication module 106 may contain a power managers 312. The power manager 312 may be configured for controlling power routing (e.g. via the electrical receptacle interface 218), components and an internal power sources 314 (e.g. in the battery compartments 226). The power manager 312 may additionally be configured to manages power operations of the communication system 102 such as for powering the communication system 102 itself, draw power from a connected peripheral communication device 110 with power sharing capabilities and/or directing power to charge a connected radio or EUD (e.g. See first configuration 702 and second configuration 704 in FIG. 7A and FIG. 7B).

In one embodiment, the communication module 106 may comprise one or more wireless module(s). According to the present example in FIG. 3A, such wireless module(s) may be a Bluetooth or near-field wireless communication component 318 in connection with a Wi-Fi / Bluetooth antenna 326 for short range wireless communication with additional devices used by the user 100 for providing a wPAN (wireless personal area network). Additionally, or alternatively the communication module 106 may in one embodiment comprise a Digital Enhanced Cordless Telecommunications (DECT) module 316. The DECT module 316 may be in connected to a dedicated DECT antenna 328 arrangement configured to operate in both a star topology or a mesh topology together with other users (e.g. Each individual other user having a similar configuration as the user 100 displayed in FIG. 1) via their equivalent communication systems.

In one embodiment, the communication module 106 may contain a user interface (UI) handler 320. The UI handler 320 may be configured for communication with a user interface 108 to provide user instructions to the communication module 106 by for example pushing a tangible control, such as a push button or toggle switch (e.g. see 224a-224c of FIG. 2A).

For example, a push-to-talk actuator module 324 may be signaled by the UI handler 320 to transmit a control-signals associated with push-to-talk (PTT) control-instruction to a transceiver module of a connected radio 110 to establish a person-to-person voice communication by transmitting a voice message using RF. Thus, when a user 100 activates the one or more of the tangible controls (e.g. 224a-224c) on the user interface 108 a Push-To-Talk (PTT), a Carrier Operated Relay (COR) and/or a Carrier Operated Switch (COS) signal (control-instruction) may be generated or triggered by the actuator module 324 and transmitted to the connected peripheral communication device 110. The control-instruction may operate by generating a control-signal that switches between logic levels (typically +5V and Ground) in the transceiver module, indicating the activation of the transmitter by activating a squelch circuit or similar in the communication device 110 thereby allowing the connected peripheral communication device 110 to transmit a audio signal.

In one embodiment, a CODEC module 322 may additionally form part of the communication module 106.

The CODEC module 332 may be used for communication with an analog peripheral communication device for transforming analog audio signals into a digital format (ADC) and vice versa (e.g. digital-to-analog (DAC) conversion) within a single unit. An analog audio signal may be a continuous electrical representation of sound waves. The analog audio signal may thus directly mirror the fluctuations in air pressure caused by sound, varying in voltage or current to correspond with the original acoustic signal.

In one embodiment, the communication module 106 may comprise a system on a chip (SoC) 330 coupled with additional electronic circuits and devices 338. The SoC 330 may be a Qualcomm QCC5181 or a Snap Dragon S7 sound chipset constraining one or more microprocessors 336, such as an MCU 302 and a DSP 304.

One example of the communication module 106 comprising a system on a chip (SoC) 330 is schematically illustrated in FIG. 2B. In the present example, the SoC 330 contains integrated hardware (HW) circuits 332 for providing specific data interface functions, such as general purpose input/output (GPIO), Analog-to-Digital signal Converters (ADC), Inter-Integrated Circuit (I2C) communication bus, "USB" controller, "COM" interface supporting RS232 serial communication, Inter-Integrated Circuit Sound (I2S) communication bus, Digital to Analog converters (DAC), Active Noice Reduction (ANR) circuits, and wireless communication module such as a Bluetooth module and a DECT module. The integrated hardware (HW) circuits 332 may be used as internal communication interfaces between the SoC 330 and the additional electronic circuits and devices 338.

In one embodiment the SoC 330 may contain a plurality of device drivers 334.

In the present example, the drivers 334 may be dedicated software components that enables communication between at least the microprocessors 336 and the hardware (HW) circuits and components 332. Other examples of drivers 334 may additionally include specific communication drivers for USB HID (Human interface device) commands and Universal Asynchronous Receiver/Transmitter (UART) drivers for communication via the COM interface and/or USB controller 310. The additional electronic circuits 332 and drivers 334 of the communication module 106 may facilitate the communication and data exchange between the different components in the communication system 102 (e.g. left connector interface 216a, right connector interface 216b, battery compartment 226, microphones (e.g. 214, 234a, 234b 236a, 236b) and speakers (e.g. 212a, 212b), user interface 108 and receptacle interface 218 etc. as displayed in FIG. 3B visualized as connection arrows in the short-dashed box 340 pointing to and from the additional electronic circuits and devices 338 block , thus enable communication between the communication module 106 to at least the hearing protection headset 104, the user interface 108 and connections to a peripheral communication devices 110. The additional electronic circuits and devices 338 may consist of;

A power supply circuitry 342 adapted to manage the connections to a plurality of power exchange lines, such as a connection to an internal power supply (e.g. an AA battery), provide power to the left connector interface 216a and right connector interface 216b and/or manage change and discharge connections to peripheral communication devices 110 with power sharing capabilities via the receptacle interface 218 (e.g. See FIG. 4A and elsewhere).

An accessory detection circuit 344 adapted to manage the connection to the left connector interface 216a and the right connector interface 216b of the headset 104 for detecting the presence of a first type of accessory device and/or a second type of accessory device, such as the boom microphone 214, and determine which of the left 216a and right connector interface 216b its connected to and the type of accessory device (e.g. boom microphone 214 or toggle switch 600 see FIG. 6A).

An IO expander circuit 346 for managing the connections and signals for a plurality of input/output devices such as for example the user interface 108 and associated tangible controls (e.g. Push bottoms 224a-224c).

A CODEC unit 348 for analog-to-digital and digital-to-analog signal conversion, utilizing for example pulse-density modulation to convert an analog audio signal into a digital format and vice versa. The unit 245 may be in direct contact with the microphones (e.g. 214, 234a, 234b 236a, 236b) and speakers (e.g. 212a, 212b) of the headset 104.

A downlead circuitry 350 for controlling and managing a wired connection to a peripheral communication device 110 (e.g. via a cable 112 and connector 220). The download circuitry 350 may be in electronic contact with the receptacle interfaces 218 and provide a plurality of connection lines (e.g. See FIG. 4A, 402a-402g) for communication with a connected peripheral communication device 110 via a connector 220 such as at least both receive (Rx) and transmit (Tx) voice signals and audio data via a transceiver module in a connected peripheral communication device 110 (e.g. see FIG. 4A, FIG. 4B and related description).

At least one antenna circuitry 352 for enabling wireless communication with a peripheral communication device 110 and/or another similar communication system 102 containing a similar communication module 106.

In one embodiment, the microprocessors 336 of the system on a chip (SoC) 330 may be configured to run embedded software and dedicated firmware. In the present example in FIG. 4B, an embedded software platform 354, preferably running on an MCU 302, may be configured to handle specific programs or control functions, visualized as modules 356a-356n. The embedded software platform 354 may contain a system core 358 comprising a real-time operating system (OS) 360b for management of hardware resources, provides common services for programs or modules 356a-356n, and ensures efficient system operation, a "State Machine" 360a for controlling high-level dataflows and execute specific actions e.g. of the modules 356a-356n by transitioning between a finite number of states based on input signals and a "Scheduler" 360c that manage and automates the execution of tasks or jobs (e.g. programs, modules or functions) in an embedded software platform 354. The system core 358 may be operably coupled and configured to control a DSP 304 unit containing a processing platform 362 adapted to execute advanced audio algorithms and functions 362a- 362j.

Examples of software modules 356a-356n running on the embedded software platform 354 may be;

A "Register read/write module" 356a for configuration of a flash memory 306 that can be programmed with the embedded software. The register read/write module 356a may be used for system configuration, firmware and software updates etc. such that the module is enabled to perform low level reconfigurations of the system on a chip (SoC) 330 and other components.

A "chip read module" 356b for obtaining coupling data by reading and passing cable chi settings stored in a cable chip 222 of a cable 112 (e.g. see FIG. 4B and elsewhere) in relation to a connected peripheral communication device 110 into the communication module 106 via the downlead circuitry 350 (e.g. and the receptacle interface 218).

A "power management" module 356c for controlling the handling power routing, power priorities and switching between power sources in contact with the power supply circuitry 342 such as the battery compartment 226 and the receptacle interface 218.

A "cable chip feature module" 356d for storing and maintaining a library or repository of available features (e.g. As described in relation to coupling data) and executing corresponding instructions and functions in accordance with the "chip read module" 356b such that the communication module 106 may perform specific actions and functions in response to the obtained coupling data.

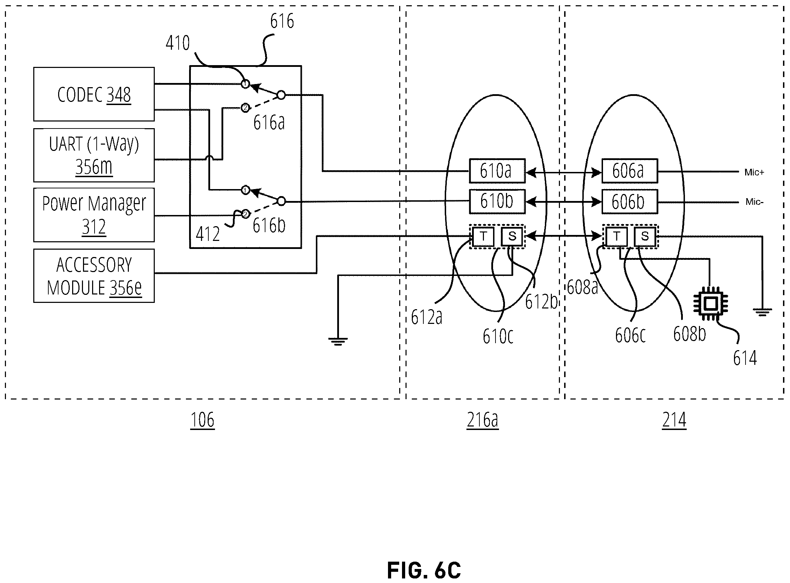

An "accessory module" 356e, configured to automatically adjust other modules 356a-356n and audio algorithms 362a- 362j in correspondence with the detection of a first type of accessory device and/or a second type of accessory device connected to the communication module 107 via the accessory detection circuitry 241 (e.g. See FIG. 6C and related description).

A "voice assistant" module 356f configured to handle, language settings, voice prompts messaging and voice instructions associated with the communication module 106 to the user 100 via the speakers 212a, 212b of the headset 104, such as device paring etc., tones, warning tones if battery low, on-connect-verification etc., and more sophisticated artificial intelligence voice assistance for a user 100 to interact using voice commands via the boom microphone 214 for example.

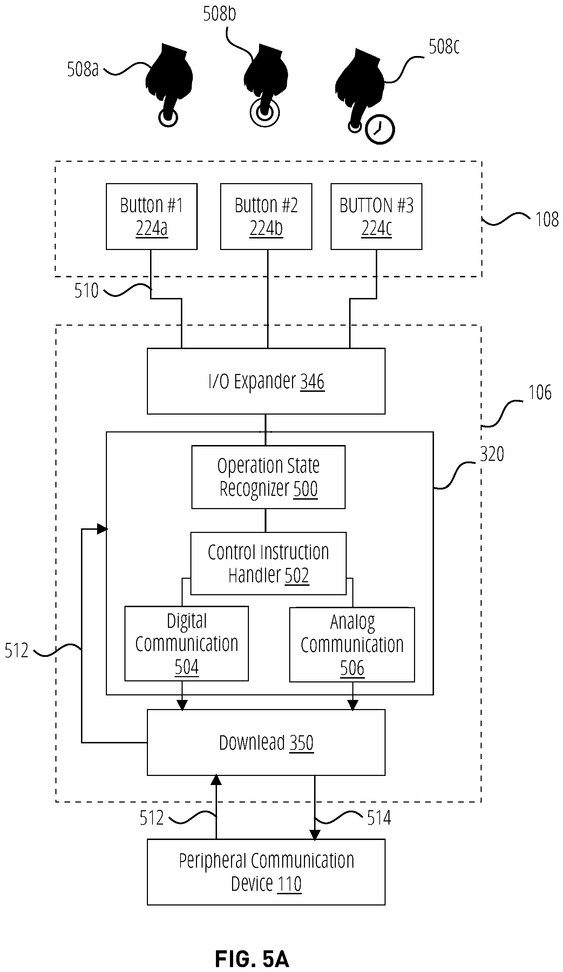

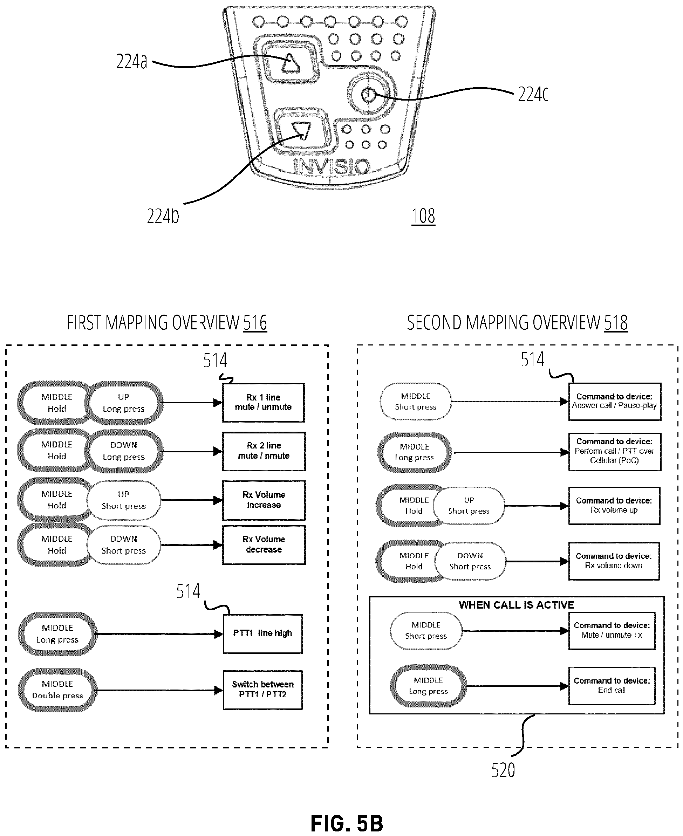

A "UI module" 356g for handling the functionalities of the user interface 108, such as user activations, operation states of the tangible controls (e.g. 224a-224c) and related control-instructions (see FIG. 5A, FIG. 5B and related description). The UI module 255g may receive electronic signals generated by a user interaction with the user interface 108 on the hearing protection headset 104 via the I/O expander circuitry 346 and may define and configure a control-instruction for an operation state automatically in accordance with both the "chip read module" 356b and the cable chip feature module" 356d as descried in more details in relation to FIG. 5A and elsewhere.

A "Audio routing" 356h module for controlling and instructing the DSP 304 how to perform audio mixing and directing audio signals to and from a connected peripheral communication device 110, between different components (e.g. 338, 330), microphones (e.g. 214, 234a, 234b 236a, 236b) and speakers (e.g. 212a, 212b) of the headset 104. The audio routing module 356h may contain and dynamically update a mixer table for controlling the audio signal flows between at least the microphones (e.g. 214, 234a, 234b 236a, 236b), the speakers (e.g. 212a, 212b) and connected peripheral communication devices 110.

A "PTT module" 356i as described in detail in relation to FIG. 2A (e.g. PTT actuator 324) and elsewhere, configured to handle and generate a Push-to-Talk (PTT) signal (PTT control-signal) to a 2-way radio (e.g. 110) used to communicate on a half-duplex communication link, that can be used for communication in both directions i.e. receive (Rx) and transmit (Tx). A PTT control-signal send to the radio 110 allows a user 100 to switch between voice transmission mode and voice reception mode within the transceiver module of the connected peripheral communication device 110. A PTT control-signal may be a DC/AC volage signal applied to the keying line of the radio (e.g. a PTT circuitry of the peripheral communication device 110) which "goes high", and a collector (e.g. also part of the PTT circuitry of the peripheral communication device 110) goes close to ground which may trigger a control/audio input line in the transceiver and switches the transmitter on allowing the peripheral communication device 110 to send a voice signal or audio data via an inbuild RF antenna system (e.g. part of the peripheral communication devices 110).

A "crosstalk module" 356j in contact with the CODEC 348 for controlling the blocking audio signals to a speaker unit e.g. left speaker units 212a or right speaker unit 212b in the same side (e.g. Left or Right) of the attached boom microphone 214 when transmitting a voice signal (e.g. Detected by the accessory detection module 356e). Thus, crosstalk refers to unintended transmission of audio signals between communication channels or circuits which is undesirable in tactical communication where sensitive or confidential messages then may be transmitted. The mechanically and electrically coupled (e.g. via a connector interface 216a, 216b) boom microphone 214 may record the acoustic vibration generated by the membrane movement of the left speaker unit 212a or the right speaker unit 212b when a sound signal is being emitted from the speaker which may accidental be transmitted via a connected peripheral communication device 110**.**

A "JSON api" module 356k, or JavaScript Object Notation Application Programming Interface, for handling data communication between third party applications, such as apps or widgets on a smartphone or tablet to interact with the communication module 106 both wired and wirelessly, such that information and functions of the communication system 102 may be displayed, visualized and controlled via a external connected touch screen or alike.

A "Mesh module" 356l for handling the connections and configuration of communication links between two or more similar communication modules 106, such as via a wireless DECT module 316 to enable pair and negotiate data exchange between the communication system 102 and one or more other similar communications communication systems 102 operating in a mesh or start topology network arrangement using the at least one antenna circuitry antenna 352.

A "UART" module 356m for handling communication and exchange of information via the universal asynchronous receiver-transmitter (UART) protocol and dedicated hardware (e.g 332 and 338) to other devices. The UART module may handle 1-way UARD connections to other devices (e.g. via the receptacle interfaces 218 and/or left connector interfaces 216a and right connector interface 216b) enabling low-data rate digital data exchanges via a single connection line (e.g. See third configuration 706 in FIG. 7C).

A "Tasks" module 356n for handling and maintaining a task list, such that the sequential order of one or more functions 362a- 362j and other modules 356a-356m may be executed in the right sequence.

Examples of audio algorithms and functions 362a- 362j running on processing platform 362 of the DSP 304 may be;

A "ANR/ANC" function 362a for performing automatic noise reduction (ANR) and/or automatic noise cancellation (ANC) inside the left and right headset cavities 210 via dedicated circuits to perform "feed-forward noise reduction" using the ambient microphones (e.g. 236a and 236b) and the speaker units (e.g. 212a, 212b) and/or "feed-backwards noise reduction" using the monitor microphones 234a, 234b and the speaker units 212a, 212b. The ANR/ANC function 362a may use one or more dedicated electronic circuits (e.g. part of the integrated hardware (HW) circuits and components 332) to generate anti-noise signals (e.g. via the speaker units 212a, 212b) that destructively interfere with ambient sound to cancel it thereby providing an improved hearing protections relative to only utilizing passive sound attenuation.

A "VOX" function 362b controlling a "voice activated transmission" configuration of the communication system 102 allowing a voice signal to be transmitted via a communication link (e.g. A radio 110) automatically when the user 100 starts speaking (e.g. Voise is detected). The VOX function 362b may signal the DSP 304 and PTT module 356i, such that the boom microphone 214 is activated/deactivated automatically and a voice signal (when detected) is transmitted via a connected peripheral communication device 110.

A "hear through" function 362d, enabling the communication system 102 to detect external sounds that the user might want to hear (e.g. via the ambient microphones 236a, 236b) which then may be processed in real-time by the DSP 304 before those external sounds are reproduced by speakers (e.g. 212a, 212b) inside the headset 104. This function providing the effect of making the headset "transparent" in terms of listening to the surroundings.

An "Active HP" function 362c, for providing active haring protection. This function co-function with the "hear through" function 362b in which ambient sound recorded by the ambient microphones 236a, 236b are allowed to be transmitted to the user 100, where the "Active HP" function 362c may limit the overall amplitude of the sound signals emitted by the speaker units 212a, 212b so as to protect the user's 100 hearing. This feature provides the user 100 with situational awareness by enabling the user 100 to hear and react to ambient external sounds, while protecting the user's hearing from overly loud sounds such as heavy machinery or a gunshot etc. which is highly advantageous when used in harsh and demanding environments. The "Active HP" 362c function may advantageously function with the sound signal received (Rx signals) via a connected peripheral communication device 110, such that audio played to the user 100 from a remote destination may also be limited in overall amplitude to protecting the user's hearing if for example another user may fire a weapon with VOX activated, causing the second user to transmit the sound of the gunshot to the first user which otherwise (e.g. without the )could cause the speaker units 212a, 212b to emit sound in a dangerous volume for the hearing ability of the user 100.

A "Echo cancelation" function 362e to remove echo sound artefacts may may appear in the communication system 103 due to acoustic feedback between the microphones (e.g. 214, 234a, 234b 236a, 236b) and the speakers (e.g. 212a, 212b).

A "Tx/Rx" function 362f for controlling audio signals and voice data received (Rx) via a connected peripheral communication device 110 and emitted to the user 100 via one or both of the speakers 212a, 212b and/or audio signals and voice data recorded (e.g. via the boom microphone 214) and Transmitted (Tx) via a connected peripheral communication device 110**.**

A "Noise reduction" function 362g for reducing noise contributions on one or more audio signals or audio data or preferably as described at least in part in US10726859 B2 (hereby incorporated by reference in its entirety).

A "scene detection" function 362h may be handling automatic scene detection also using the "AI engine" or other functions, such that ambient scene, like rainy weather, sitting in a helicopter, quiet surroundings, high windspeeds, heavy machinegun fire, etc. may be recognized based on audio input via ambient microphones (e.g. 236a, 236b) on a hearing protection headset 104 such that other modules 356a-356n and functions 362a- 362j may be adjusted to optimize the performance of the communication system 102 in different harsh environments (e.g. Dessert environment, urban environment, forest/jungle environment etc.).

A "auto gain" function 362i, for performing environmental adaption of sound signals, such that in case of a high noise environment (e.g. recorded via the 236a, 236b) may cause the volume of sound signals emitted via the speaker units 212a, 212b to be increased (i.e. within the limit of the "Active HP" function 362c) and vice versa, such that the user 100 are always able to hear voice messages received clearly and undistorted even in harsh and demanding environment with high background noise or preferably as described at least in part in US11386879 B2 (hereby incorporated by reference in its entirety).

A "AI engine" function 362j enabling trained neural network models to be applied to perform advanced machine learning processing tasks one the different voice signals and audio data such as one or more of speech recognition, voice-to-text, image classification, enhanced situational awareness, 3D directional sound processing, advanced signal processing, active noise cancellation.

It should be understood that modules 356a-356n and functions 362a-362j are provided as examples of functionality and operation of the communication system 102 as perceived by the user 100. Functionalities may be handled and executed by the communication module 106 in a mix between at least parts of one of more modules 356a-356n and functions 362a-362j and other components of the communication systems 102. The provided examples are non-exhaustive and both fewer or more modules 356a-356n and functions 362a-362j may advantageously form part of the communication module 106 in some embodiments.

### Coupling data

In one embodiment, the communication module 106 (e.g. see FIG. 3A, FIG. 3B) may be configured to connect different types of peripheral communication devices, such as at least a first type of peripheral communication device and a second type of peripheral communication device and in response to identifying the connected peripheral communication devices type, adapt the communication scheme (i.e. data exchange protocol), such that the communication system 102, comprising the communication module 106, may transmit a first control-signal comprising one or more first control instruction(s) of and to the first type of peripheral communication device, and transmit a second control-signal comprising one or more second control instruction(s) of and to the second type of peripheral communication device. Wherein the control-instruction(s) of the first type and second type of peripheral communication device(s) respectively is defined when the communication module 107 receives first or second coupling data.

In a preferred embodiment, the communication module 106 may be configured to receive first coupling data and/or second coupling data from a socket (e.g. receptacle interface 218) in contact with the communication module 106 (e.g. via the downlead circuitry 350) and/or from a plug (e.g. connector 220) being connected to the communication module 106.

Preferably, where in one embodiment, the socket 218 is configured to recognize the type of device being connected to the socket 218, more preferably where the plug 220 comprises a microchip (e.g. cable chip 222) comprising the first coupling data or the second coupling data.

In some embodiment, wherein the first coupling data comprises said first control-instruction(s), and/or wherein the second coupling data comprises said second control-instruction(s). This may be advantageous as both the first and second control-instruction(s) may be directly programmed into the memory of the cable chip 222, such that the communication module 106 may read the control-instructions directly from the cable chip 222 associates with the connected peripheral communication device and configure the functionality of the communication module and hearing protection headset 104 accordantly.

In one preferred embodiment first and second coupling data may advantageously be obtained via a cable chip 222 (e.g. See FIG. 2A and elsewhere). The cable chip 222 (and/or an associated memory of the chip like a serial EEPROM), may allow a set of settings to be stored. When the memory is read by the communication module 106 (e.g. via the receptacle interface 218 and downlead circuitry 350), the settings may be transferred into the microprocessors 336 (e.g. MCU 302 and DSP 304) and distributed across the embedded software platform 354 and processing platform 362 to the appropriate sections of the code (e.g. modules 356a-356n and functions 362a-362j) and/or peripheral internal components and units etc. (e.g. Microphones 214, 234a. 234b, 236a, 236b. Speakers 212a, 212b, interfaces 218, 216a, 216b etc.).

In one embodiment the stored information/data of the cable chip 222 may e.g. be organized as a list of "feature calls", also denoted "feature requests", followed by specific settings and e.g. other data respectively associated with the requested feature (i.e. the feature request).

In one embodiment, the cable chip 222 may store feature-requests (of/for the connected device type) that when obtained by communication module 106 may cause the relevant processor(s) (e.g. MCU 302 and DSP 304) to execute instructions stored in the memory 306 of the communication module 106 corresponding to and/or carrying out the specific functionality associated with a requested "feature". For example, the requested features may be applied by the communication module 106 (e.g. via the cable feature module 356d) using a specific set of settings, values and/or data transferred from the cable chip 222 for that particular feature.

For example, a feature-request could be "1000 - COM Rx Audio line" requesting feature "1000" which may be relevant in relation to setting up a configuration in the communication module 106 related to signal routing of received audio communication from a radio (e.g. A peripheral communication device 110). The "1000 - COM Rx Audio Line" feature-request may additionally contain a list of settings which could be transferred and applied by the communication module 106 when configuring the feature. Examples of settings could be: set "Physical Audio line" to "RX1", set "Gain" to "2" [dB] and set "Max voltage for Rx Line" to "14 V" etc., Thus, in one embodiment the cable chip 222 information may be used to instruct the communication modules 106 about the required functionality (features) and how exactly the functionality should be applied (settings). For example, when a feature-request is read by the communication module 106 the one or more processors (e.g. MCU 302 and DSP 304) may execute the code associated with the requested functionality and perform (e.g. see FIG. 3A and FIG. 3B and related description) the configuration of the communication module 107 based on the specific settings, such as:
Setup peripheral directly, including:
   - interface type, Gain levels, UI instructions, etc.
Setup DSP 304 (e.g. see FIG. 3A and FIG. 3B) directly, including:
   - Audio algorithms, static audio flow, etc.
Setup MCU 302 (e.g. see FIG. 3A and FIG. 3B) directly, including:
   - Data protocol etc., software functions and communication.
Stored in MCU 302 for more dynamic behavior, including:
   - all settings that change depending on UI 109 or other events from audio or other cables 112 /headsets 104.

Thus, the functionalities can be static or processed dynamically depending on events (audio events, button push, timer event, protocol event).

A cable chip 135 in a cable 113 may in one embodiment have an arbitrary number of features ranging from 4 features for a simple setup to 20+ features for complex cases. In one embodiment, a feature may be a group of 5-20 settings (but can be everything from 0 - 50 settings or more).

In some embodiment, a feature may be grouped into a number of categories associated with the requested configuration of the connection interface; examples of such categories are:
Audio Interfacing;
   - Defines audio interface, impedance, gain, etc.
Device type / Protocol Interfacing;
   - Which may define the connected device type, such as digital or analog and specify required communication protocols UART, USB, PTT open collector, Pulsing interface e.g..
Control functions;
   - Linking what function should be enabled based on event, such as VOX or VAD
UI definitions;
   - Which may define activation states, i.e. Short/Long press tangible controls (e.g. 224a-224c) plus key combos (i.e. combination of different simultaneous button presses) on the user interface 108 and UI definitions (e.g. control-instruction(s))
Audio algorithm;
   - Which may define applicable audio algorithms and the parameters to control the algorithms
Audio Routing;
   - Which may define the flow of the audio including multi headset setup (e.g. combining the headset 104 with a pair of in-ear earbuds for dual hearing protection), Crossbanding/Relay between two or more radio transceivers and side tone to the user 100

In summary, the communication modules 106 may be configured on the basis of coupling data, such as information stored in the interface of connected cables 112 (e.g. cable chip 222) specifying configurations details in relation to a connected peripheral communication device type such as an analog radio (e.g. 708 See FIG. 7A) or a digital tactical EUD (e.g. 710 see FIG. 7B).

It is advantageous to obtain coupling data via a cable chip 222 (e.g. information/data), as the communication module 106 is configured for at communication system 102 to be use in harsh and demanding environment to provide both hearing protection and clear and undistorted communication. Typically, different types of peripheral communication devices are used by police and military personal, spanning across modern digital devices like tactical smart phones communication via a digital USB protocol and legacy reliable analog LMR devices communication by exchanging analog control-signals. By obtaining coupling data via a cable chip 222 upon connection of the peripheral communication device, the communication module 106 may configure the communication scheme according to the peripheral communication device type (e.g. analog or digital communication) and initiate the correct data signal exchange scheme. Thus, the coupling data via cable chip 222 read provide the advantage of configuring the communication system 102 to switch back and forth between different data exchange schemes, such that the communication module 106 can be configured to connect to an analog communication device (e.g. A first type of peripheral communication device) or a digital communication device (e.g. A second types of peripheral communication device.

Thus, in one embodiment, the one or more control-instructions of said first control-instruction(s) is defined as a first communication protocol or wherein the one or more control-instructions of said second control-instruction(s) is defined as a second communication protocol

Digital "handshakes" known from device type identification and host/devices negotiation via a USB protocol are simply not possible to rely on when the communication module 106 is configured to communication with legacy analog peripheral communication devices, as exchange of detailed data packages are not supported via analog signals. In one embodiment, the cable chip 222 may enable the peripheral communication device to specify its type. This way, the communication module 106 may adapt the data exchange scheme thereby making it possible for the communication system 102 to changes between different data exchange schemes.

In some embodiments, the coupling data may be obtained via a digital initial "handshake". A Digital handshake may be a "USB handshake" comprising coupling data transfers like "Get Device Descriptor", "Get Configuration Descriptor" and "Set Address", (e.g. as defined USB 2.0 specification). In one embodiment, the communication module 106 may be configured as a "USB peripheral".

In another embodiment the connected peripheral communication device 110 may be configured as a " USB host" that manages the data exchange.

It is particular advantageous to use digital handshakes for the initial negotiation of the roles of " USB host" and "USB peripheral" when the communication system 102 may primarily communicate with digital USB type peripheral communication devices 110 (e.g. Second type of peripheral communication device) as coupling data may be obtained directly from a memory component or other related circuitries of the peripheral communication device directly without the need for a dedicated cable chip 222.

### Electronic receptacle interface

In one embodiment the communication system 102 may comprise a receptacle interface 218 configured to connect a first type of peripheral communication device and/or a second type of peripheral communication device via a cable 112 and a connector 220 having a plurality of connector pins 404a-404g , and exchange electronic signals via the plurality of connector pins, wherein the electronic signals comprise at lease audio data for connecting a peripheral communication device 110 via a cable 112 and a connector 220.

FIG. 4A schematically illustrates an example of the electronic receptacle interface 218 of the communication system 102 according to the disclosure. FIG. 4A show a front view of the electronic receptacle interface 218 connected to the communication module 106 (e.g. see FIG. 2A) and configured to connect a first type of peripheral communication device and/or a second type of peripheral communication device via a cable 112 and a connector 220 (e.g. See FIG. 4B). The electronic receptacle interface 218 may have a cylindrical metal housing 400 adapted to mechanical connect to a complementary mating connector (i.e. the connector 220) along a connection axis and adapted to provide a mechanical locking mechanism when coupled to a connector 220, such a push-pull locking or breakaway function. The electronic receptacle interface 218 may be an ODU AMC type rugged miniature connector interface for harsh environments. The receptacle interface 218 may be waterproof both when mated (e.g. when physically coupled to the connector 220) and unmated. The electronic receptable receptacle interface 218 may comprise a plurality of terminals 402a-402g configured to individually couple with corresponding plurality of connector pins 404a-404g in the connector 220 (e.g. see FIG. 4B), such that electronic signals (e.g. Control-signals and control-instructions etc.) may be exchanged between the plurality of terminals 402a-402g and connector pins 404a-404g when mated. The electronical receptacle interface 218 may preferably have 7 individual terminals such as a first terminal 402a, a second terminal 402b, a third terminal 402c, a fourth terminal 402d, a fifth terminal 402e, a sixth terminal 402f and a seventh terminal 402g all being communicatively coupled to the communication module 106, such as via the downlead circuitry 350 and the power supply circuitry 342 (e.g. see FIG. 3B), for exchanging electronic signals comprising at lease audio data, control-instructions and/or power between the communication system 103 and a first- and second type of connected peripheral communication device 110 via a cable 112. By limiting the supported number of individual connector pins 404a-404g to a total number of 7 provides the advantage that the overall formfactor of the connector 220 and receptacle interface 218 may be relatively small and compact, such that its cheaper to produce and may fit better into a hearing protection headset 104 for example as the size is smaller. Additionally, 7 individual terminal 402a-402g provide the necessary connections (wires) to support both analog type peripheral communication devices and digital type peripheral communication devices. Also, by supporting a total number of 7 pins in the connector requires the cable only to contain or accommodate a low number a individual wires (i.e. 7 in total), making the cable relatively thin and flexible. This is highly preferable for a user operating in harsh and demanding environments as cables are required to be mounted and routed on the west and equipment of a soldier. A relatively thin and flexible cable provides the advantages that it's easier to handle, mount and route on the vest or equipment thereby not obstructing movement or the reach to other necessary equipment.

FIG. 4B schematically illustrates an example of a connector 220 having a plurality of connector pins 404a-404g and configured to connect a peripheral communication device 110 to the communication system 102. The connector 220 may additionally be an ODU AMC type rugged miniature connector interface for harsh environments. The connector 220 may have a plurality of connector pins 404a-404g for exchanging electronic signals between the communication module 106 and a connected peripheral communication device 110. Each connector pin 404a-404g may extend along individual wires inside the cable 112 and connect to respective circuit boards, interfaces and processors of the peripheral communication device. The connector 220 may preferably comprise a cable chip 222 for obtaining coupling data as described in detail elsewhere for providing at least device type information related to the connected peripheral communication device 110. The connector 220 may be embodied as described at least in part in EP2942837A1 (hereby incorporated by reference in its entirety).

In one embodiment, the communication module 106 in contact with the electronic receptacle interface 218 may be configured to switch between exchanging the electronic signals with the connected peripheral communication device 110, via the plurality of connector pins 404a-404g (i.e. and terminals 402a-402g), according to a first data exchange mode, such as an analog communication mode, and a second data exchange mode, such as an digital communication mode, wherein the first data exchange mode is applied when the electronic receptable user interface 108 is connected to a first type of peripheral communication device, such as an analog radio (e.g. A Motorola XTS 5000 radio 708 See FIG. 7A), and the second data exchange mode is applied when the electronic receptacle interface 218 is connected to a second type of peripheral communication device, such as an EUD ( e.g. A Samsung Galaxy XCover6 Pro Tactical Edition EUD 710 see FIG. 7B. It is advantageous that communication module 106 may be able to switch between a first data exchange mode and a second data exchange mode via the receptacle interface 218, as this enables the communication system 102 to dynamically adapt itself to communicate with different types of peripheral communication devices, such as a legacy analog LMR and a new modern tactical smartphone in a wired manner via a single socket (e.g. Receptacle interface 218). This provides a communication system 102 to be used in harsh and demanding environments which can connect and operate a wide range of different communication types (e.g. At least digitally and in an analog manner) via a single "plug and play" interface making connections unambiguous and ease of the user to operate. By providing a communication systems 102 comprising an agnostic data exchange interface (e.g. Receptacle interfaces 218), which automatically switches between first data exchange and a second data exchange mode according to the connected device type enable the user 100 in a harsh environment to have clear and undistorted communication while maintaining focus on the mission and tasks ad hand rather than struggling with compatibility issues when connecting a peripheral communication device 110.

In a preferred embodiment, the communication system 102 may be embodied as a hearing protection headset 104, such that a single device (i.e. the hearing protection headset 104) may automatically switch its communication mode dependent on the connected peripheral communication device 110, such that a user 100 may simply grab the headset 104 and any type of communication device from a shelf, plug the cable 112 to the peripheral communication device intro the headset 104 and have instantaneous clear and undistorted communication capabilities not matter what type of peripheral communication device used.

In one embodiment, the terminals 402a -402g of the receptacle interface 218 may be configured such that: The first terminal 402a may be configured for exchanging electronic signals with a sense connector pin 404a for obtaining coupling data associated with a connected peripheral communication device 110 such that the receptacle interface 218 may be configured to recognize the type of device being connected to the electronic receptacle interface 218. The second terminal 402b may be configured for exchanging electronic signals with an audio transmission connector pin 404b adapted for transmitting voice data (e.g. from the boom microphone 214) to a connected peripheral communication device 110, when the communication module 106 is exchanging the electronic signals according to the first data exchange mode. The third terminal 402c may be configured for exchanging electronic signals with a first data connector pin 404c, and the fourth terminal 402d may be configured for exchanging electronic signals with a second data connector pin 404d according to a first data exchange mode when the electronic receptacle interface 218 is connected to a first type of peripheral communication device and a second data exchange mode when the electronic receptacle is connected to a second type of peripheral communication device. The fifth terminal 402e may be configured for at least exchanging electronic signals with a PTT connector pin 404e which may be adapted for transmitting a PTT signal to a first type of peripheral communication device, when the communication module is exchanging the electronic signals according to the first data exchange mode. The sixth terminal 402f may be configured for exchanging electronic signals with a power connector pin 404f for directing power to or from the first type of peripheral communication device or from the second type of peripheral communication device. The seventh terminal 402g may be configured for a ground connector pin 404g, for connecting to common electronic ground.

In one embodiment, The communication module 106 may be configured to switch between exchanging the electronic signals, via the plurality of connector pins (e.g. 404a-404g), according to a first data exchange mode and a second data exchange mode.

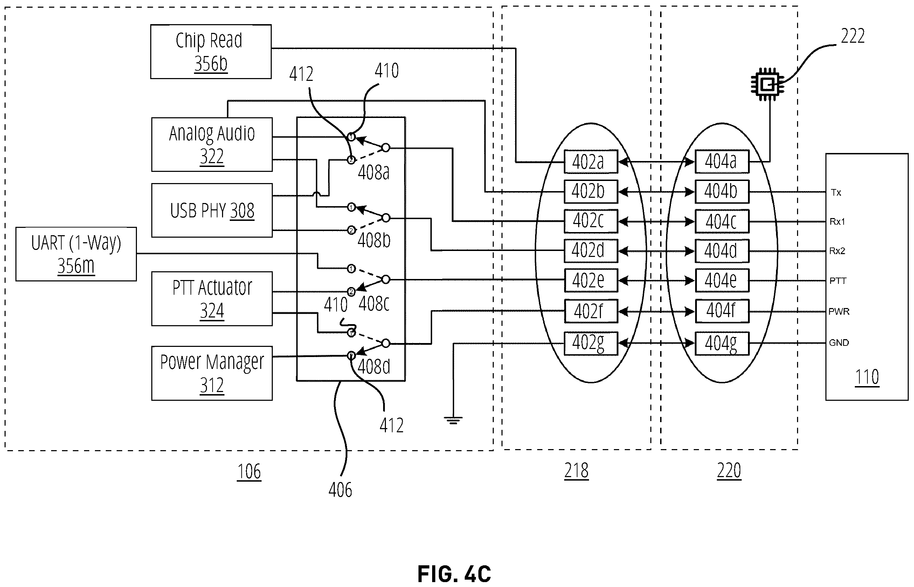

FIG. 4C schematically illustrates an example of a communication module 106 comprising an electronic receptacle interface 218 and a multiplexing (MUX) circuitry 406 according to at least embodiment of the disclosure. FIG. 4C show an example of a electronic signal routing architecture of the electronic receptacle interface 218 and communication module 106 connected to a peripheral communication devices 110 being a first type of peripheral communication device. The first type of peripheral communication device may be connected to the receptacle interface 218 via a connector 220 such that the plurality of connector pins 404a-404g (e.g. And corresponding terminals 402a-402g) may electronically couple to the communication module 106 via one or more of the additional electronic circuits and devices 338 (e.g. see FIG. 3B) and a multiplexing (MUX) circuitry 406 comprising a plurality of switching circuits 408a-408d, using logic gates or transistor-relays, and configured electronically switch between different signal exchange routes. Thus, one or more terminals 402a-402g may individually be connected to a switching circuit 408a-408d of the communication module 106.

In the example displayed in FIG. 4C, the first terminal 402a may exchange electronic signals with the sense connector pin 404a in contact with the cable chip 222 comprising coupling data. The first terminal 402a may be connected to the downlead circuitry 350 of the communication module 106 such that coupling data may be obtained via the chip read module 356b specifying at least that the connected peripheral communication device is a first type of peripheral communication device, such as an analog type (e.g. a Motorola XTS 5000 radio 708 see FIG. 7A). The identification of the communication device type may cause the one or more of the switching circuits 408a-408d to change their state individually or collectively. The chip read module 356b may read the coupling data via the cable chip 222 immediately upon connection between the first terminal 402a and the sense connector pin 404a and cause the multiplexing (MUX) circuitry 406 to adjust the switching circuits 408a-408d in accordance with the recognized type of peripheral communication device being connected to the electronic receptacle interfaces 218. The third terminal 402c may exchanging electronic signals with a first data connector pin 404c of the connector 220. As displayed in FIG. 4C the third terminal 402c may connect to the first switching circuit 408a of the multiplexing (MUX) circuitry 406. The first switching circuit 408a may be configured to change the signal routing, such that the third terminal 402c may exchanging electronic signals with the downlead circuitry 350 eighter via the analog audio input/output logics 322 or a USB PHY 308 that may implement the physical layer functions of the USB (Universal Serial Bus) data exchange protocol. The USB PHY 308 (e.g. See FIG. 3A) may thus serve as the interface between the USB controller 310 (e.g. of the communication module 106) and the physical medium (e.g. the terminals 402a-402g and connector pins 404a-404g). In the example illustrated in FIG. 4C, the fourth terminal 402d may exchanging electronic signals with the second data connector pin 404d of the connector 220. The fourth terminal 402d connects to the second switching circuit 408b of the multiplexing (MUX) circuitry 406. The second switching circuit 408b may, similar to the first switching circuit 408a, be configured to change the signal routing, such that the fourth terminal 402d may exchange electronic signals with the communication module 106 eighter via the analog audio input/output logics 322 or the USB PHY 308. The first- 408a and second switching circuity 408b may advantageously be synchronized, such that they change between the same states simultaneously. This enables the communication system 102 to switch between exchanging electronic signals in a first way where the first data connector pin 404c and the second data connector pin 404d are configured solely to receive audio data when the communication module 106 is operating in the first data exchange mode (e.g. first- 408a and second switching circuit 408b both in first state 410, as illustrated in FIG. 4C indicated with a black solid arrow), and in a second way, where the first data connector pin 404c and the second data connector pin 404d are configured for exchanging both audio data and non-audio data when the communication module 107 is operating in the second data exchange mode (e.g. first- 408a and second switching circuit 408b both in second state 412, opposite to the first state 410). The switching of the first switching circuit 408a and the second switching circuit 408b may advantageously be performed based on the coupling data, such that the first data connector pin 404c and second data connector pin 404d may be shifted between;

Either, solely receiving analog audio signals from the connected peripheral communication device 110 (e.g. such as a Motorola XTS 5000 radio 708 see FIG. 7A) to be played to the 100 through the right speaker units 212b in the left earcup 200a and/or the right speaker unit 212b in the right earcup 200b of the hearing protection headset 104. Thus enables the Left speaker unit 212a and the right speaker unit 212b to receive stereo audio signals from the radio 110 and play the audio signals to the user 100. In other words, in the first data exchange mode the first terminal 402a and second terminal 402b is configured as analog stereo audio input terminals "Rx1" and "Rx2" to receive audio signals).

Or, exchange digital data packages between communication module 106 and the connected peripheral communication device 110 (e.g. such as a Samsung Galaxy XCover6 Pro Tactical Edition EUD 710 see FIG. 7B) according to a USB protocol, such as USB HID commands, USB Audio data using isochronous transfers, interrupt transfers, and control transfers etc. Thus, the first terminal 402a and second terminal 402b may be configured as the USB differential transmission pair (D+ and D-) for exchange of digital data packages (e.g. USB transaction) being electronic signals as a sequence of bits or binary states (e.g. "0" and "1"). When the communication module 106 is configured according to the second data exchange mode and may for example exchange electronic signals according to low speed/full speed USB bus scheme, a differential '1' may be transmitted by pulling D+ over 2.8V with a 15K ohm resistor pulled to ground and D- under 0.3V with a 1.5K ohm resistor pulled to 3.6V. A differential '0' on the other hand is a D- greater than 2.8V and a D+ less than 0.3V with the same appropriate pull down/up resistors.

In one embodiment, audio data comprises analog audio signals when the communication module 106 is exchanging the electronic signals according to the first data exchange mode, and
digital audio data packages, preferably according to USB protocol, a UART protocol, or an EtherNet/IP protocol, when the communication module 106 is exchanging the electronic signals according to the second data exchange mode.

In one embodiment, non-audio data comprise digital non-audio data packages, preferably according to USB protocol, a UART protocol, or an EtherNet/IP protocol, such as video data, control instructions, commands, instructions, metadata. GPS coordinates, images etc.

Referring to the example In FIG. 4C, the fifth terminal 402e may exchanging electronic signals with a PTT connector pin 404e of the connector 220 in contact with a PTT circuitry in first type of peripheral communication device (e.g. the Motorola XTS 5000 radio 708 see FIG. 7A). The fifth terminal 402e may connect to the third switching circuit 408c of the multiplexing (MUX) circuitry 406. The third first switching circuit 408a may be configured to change the signal routing, such that the fifth terminal 402e may exchanging electronic signals with the communication module 106 eighter via the PTT actuator 324 or a UART interface 384 used for a half-duplex or one-wire UART data exchange protocol (e.g. 1-way UART), enabling low bit rate communication between the communication module 106 and the connected peripheral communication device 110. In the example illustrated in FIG. 4C, the third switching circuit 408c is positioned in the second states 412, connecting the PTT actuator 324 and related functionality of the communication system 102 to the PTT connector pin 404e enabling the communication module 106 to key the connected first type of peripheral communication device using a Push-to-Talk (PTT) signal (e.g. a voltage signal) as describe elsewhere. The position (i.e. State) of the third switching circuit 408c may advantageously be controlled according to the obtained coupling data, such that the state may be changed in response to connecting a different device type (e.g. A first type of peripheral communication device or a second type of peripheral communication device). The state of the third switching circuit 408c may be changed to the first state 410 (e.g. 1-way UART) when, for example, connected to a PTT control unit 712 (e.g. see FIG. 7C) such as an INVISIO V60 control unit as described in relation to the third configuration 706 displayed in FIG. 7C, whereas a Push-to-Talk (PTT) signal may be send as digital control-instruction from the communication module 106 using the UART protocol to the PTT control unit 712, which may interpret the control signals and interact with devices (e.g. The Persistent systems wave relay MPUS 5 radio 714 and Thales AN/PRC-148 JEM radio 716) connected to the PTT control unit 712 (e.g. see FIG. 7C and description thereof).

In FIG. 4C, the sixth terminal 403f may exchanging electronic signals with a power connector pin 404f of the connector 220 in contact with a battery in first type of peripheral communication device (e.g. The Motorola XTS 5000 radio 708). The sixth terminal 402f may connect to the fourth switching circuit 408d of the multiplexing (MUX) circuitry 406. The fourth switching circuit 408d may be configured to change the signal routing, such that the sixth terminal 402f may exchanging electronic signals with either the power manager 312 or the PTT actuator 324 of the communication module 106 for providing an additional PTT line option (e.g. When the fourth switching circuit 408d in first state 410) or exchanging electronic signals with the power supply 342 and the power manager 312 (e.g. The fourth switching circuit 408d in second state 412. In the example illustrated in FIG. 4CC, the fourth switching circuit 408d is positioned in the second state 412, connecting the power manager 312 and related functionality of the communication system 102 to the sixth terminal 402f and power connector pin 404f. The state of the third switching circuit 408c may advantageously be controlled according to the obtained coupling data, such that the state may be changed in response to coupling data specifying desired power routing such that the communication module 106 may draw power from the connected peripheral communication device, such as the battery in the Motorola XTS 5000 radio for powering the entire communication system 102 or oppositely provide power to the connected peripheral communication device 110 is case the connected peripheral communication device 110 is adapted to receive power like in case of a USB type device (e.g. such as a Samsung Galaxy XCover6 Pro Tactical Edition EUD 710 see FIG. 7B) whereby the sixth terminal 402f may be used to exchange a VBUS voltage of 4.40V and up to a maximum voltage of 5.25V from an internal power source (e.g. one or more batteries in the battery compartments 226 or the power source of the peripheral communication device). Coupling data may in one scenario specify that the communication system 102 should utilize internal power (e.g. one or more batteries in the battery compartment 226), whereas the fourth switching circuit 408d may change state (e.g. Second state 412) enabling a second PTT line signal available to be used for exchange of electronic signals if supported by the connected peripheral communication device (e.g. wired to the correct circuitries).

The seventh terminal 402g may be exchanging electronic signals with a ground connector pin 404g of the connector 220. The seventh terminal 403g may be connected to ground being a common reference level defining 0 voltage between at least the peripheral communication device 110 and the communication system 102.

### User interface and tangible control elements

In one embodiment, the user interface 108 of the communication system 102 may comprise tangible controls (e.g. 224a-224c) that are manually operably by the user 100. FIG. 4A displays a schematic example of a user interface 108 comprising three tangible controls in the form of buttons (e.g. see FIG. 2A, 224a-224c). It may be particularly advantageous to have tangible controls on the user interface 108 when operated by a user 100 in a harsh and demanding environment, as the tangible controls should be easy to operate by the user 100 under difficult and stressful circumstances, even while wearing thick gloves or in darkness. Thus, the tangible control may have a structure and design to enable unambiguous manually operation (e.g. distinct protrusion, placement and/or texture). By having tangible controls designed as a keypad with buttons (e.g. 224a-224c) as protruding structures extending from the base positioned in a distinct pattern, enables the user 100 to locate the correct button just by the touch and feel of the button (e.g. 224a-224c) and its relative position with respect to other buttons thereby providing unambiguous manual operation. In a preferred embodiment, the tangible controls are rugged and durable, such that they may withstand rough handling, which may occur in harsh conditions, such as being covered in mud, survive a moderate impact against the ground or an obstacle etc. while still be operational.

Each of the tangible control elements (e.g. 224a-224c) may support at least one operation state and preferably more the one operation state, such as a short-press state 508a, a double-press state 508b and a long-press state 508c, as each of the operation states (e.g. 508a-508c) may be programmed (i.e. automatically configured) to enable the communication module 106 to perform different actions. This is advantageous, as fewer buttons are required to enable the user 100 to perform a wider range of user interactions for controlling the communication system 102.

The user interface 108 may be in contact with the communication module 106, such that a user interaction such as pushing a button, may cause the generation of one or more electronic signals 510 to be processed by the communication module 106. The user interface 108 may interface the communication module 106 via one or more of the additional electronic circuits and devices 338 such as the IO expander circuitry 346 thereby enabling the embedded software platform 354 and processing platform 362 of the microprocessors 336 to process and handle the response (e.g. Electronic signals 510) to one or more user interactions causing the activation of an operation state (e.g. 508a-508c) of one or more control elements (e.g. 224a-224c). An operation state recognizer module 500, which may form part of the UI module 356g, may be adapted to recognize a specific activation associated with each of the control elements 224a-224c. The operation state recognizer module 500 may be able to recognize at least of; a short-press state 508a, defined as a user interaction with the tactile control (e.g. 224a-224c) for less than 200 ms, a double-press state 508b, defined as an user interaction with the tactile control (e.g. 224a-224c) for less than 200 ms twice within 400 ms, and/or a long-press state 508c or a press-and-hold state 508c, wherein the long-press state 508c is defined as an interaction with the tactile control for more than 250 ms. The operation state recognizer module 500 man additionally be adapted to recognize so called "key combos", which refers to combinations of two or more user interactions (e.g. 508a-508c) being performed on two or more tangible controls (e.g. 224a-224c) at the same time.

The electronic signal 510 associated with the identified operation state (e.g. 508a-508c) may be processed by a control instruction handler 502 configured to automatically define and configure a specific control-instruction 514 for at least one identified operation state (e.g. 508a-508c) thereby enabling the communication module 106 to generate and transmit one or more control-instructions 514 using a first control-signal or a second control-signal to a connected peripheral communication device 110 depending on the device type (e.g. First mapping overview 516 and second mapping overviews 518 in FIG. 5B).

In a preferred embodiment, the control instruction handler 502 may automatically define and configure a specific control-instruction 514 for an identified operation state (e.g.508a-508c) in response to the communication module 106 receiving coupling data 512 associated with the type of peripheral communication device 110 (e.g. Coupling data 512 specifying a first type of peripheral communication device or a second type of peripheral communication device. This is highly advantageous, as the specific control-instruction 514 may be programmed and stored in the communication modules 106 and/or in a cable chip 222 such that the different operation states (e.g. 508a-508c) of the tangible controls (e.g. 224a-224c) may be configured automatically according to the connected peripheral communication devices 110 type, thereby enabling the communication systems 102 to interact and control a wide range of connected peripheral communication device types using the same user interfaces 108, even though the different peripheral communication device types may be controlled by and interacted with the communication systems 102 using different signal exchange schemes, such as for example digital data packages or analog signals. The tangible controls (e.g. 224a-224c) may thus be automatically programmed to execute different functions when activated causing the communication modules 106 to transmit different kind of control-signals (e.g. digital data packages or analog signals) dependent on the connected peripheral communication device type.

A peripheral communication device 110 may preferably be connected to the communication module 106 via one or more of the additional electronic circuits and devices 338 such as the downlead circuitry 350 for wired connections as described in relation to FIG. 4A-FIG. 4C and elsewhere. Alternatively, or additionally, a peripheral communication device 110 may be connected to the communication module 106 via the antenna circuitry 352 for wireless connections. The communication module 106 may receive coupling data 512 associated with a connected peripheral communication device 110, such as reading a cable chip 222 and pass the information to the embedded software platform 354 and processing platform 362 of the microprocessors 336 (e.g. see FIG. 3B) such that a specific control-instruction 514 may be configured for a specific operation state (e.g. ) or key combo. When a specific control-instruction 514 may be defined for at least one specific operation state (e.g. 508a-508c), the communication modules 106 may be configured to transmit one or more control-instruction(s) to a peripheral communication device 110 using a control-signal that correspond with the device type (such as digital data packages for or analog signals). Preferably, the control-signals may be defined according to specific communication protocol such as a USB communication protocol or an analog communication protocol. Advantageously, the control-signal comprise a control-instruction 514 defined to control a transceiver-module of a connected peripheral communication devices 110 to establish person-to-person voice communication via the hearing protection headsets 104. In other words, the users 100 may for be able to transmit a voice message recorded by the headset 104 via a connected peripheral communication device 110, by pressing a button on a user interfaces 108 independent of if the peripheral communication device 110 is a legacy analog land mobile radio (such as a Motorola XTS 5000 radio 708 see FIG. 7A) or an modern digital tactical smartphone (such as a Samsung Galaxy XCover6 Pro Tactical Edition EUD 710 see FIG. 7B). This is highly advantageous, as the user 100 in a harsh and demanding environment may only need to operate one single user interface 108 in order to establish clear and undistorted person-to-person voice communication, making communication easy and intuitive regardless of the communication device type. The user 100 does not need to care about device types in a stressful situation, as the communication systems 102 according to the disclosure provides plug and play functionality for the users 100 to operate and control any type of peripheral communication device 110 via the tangible controls (e.g. 224a-224c) on a user interfaces 108. When users operate in harsh and demanding environments, radios and other communication devices are often placed out or reach for immediate interactions, such as on the back of a soldier or in a pouch making remote operation of the communication devices highly desirable, the communication system 102 according to the disclosure provide a ease-of-use solution for a user 100 in demanding environments to stay focused on the task or mission while facilitating clear and undistorted communication via any type of peripheral communication device 110 by enabling control of the peripheral communication device from an intuitive and automatic configurable user interfaces 108.

In one embodiment, coupling data 512 may specify that connected peripheral communication device 110 is an analog communication device type (e.g. A first type of peripheral communication device) which may cause the communication module 106 to define a control instruction for a specific operation state via an analog communication 415 scheme, such as a CODEC to generate analog control-signals and control-instructions 514, such as contentious voltage signals, transmitted to the peripheral communication device 110. In another embodiment coupling data 512 may specify that the connected peripheral communication device 110 is a digital communication device type (e.g. A second type of peripheral communication device) which may cause the communication module 106 to define a control-instruction 514 for a specific operation state (e.g. 508a-508c) using a digital communication 504 scheme, such as a USB protocol to generate digital control-signal(s) and control-instruction(s) 514 , such as USB HID commands transmitted to the peripheral communication device 110.

In one embodiment, the communication system 102 may be configured such that each of the tangible controls (e.g. 224a-224c) may have one or more specific operation states (e.g. 508a-508c) each automatically defining and configuring a specific control-instruction for a connected peripheral communication device 110, depending on the device type. FIG. 5B schematically illustrates an example of the mapping between the tangible control elements (e.g. 224a-224c), specific operation states (e.g. 508a-508c) and associated control-instructions 514 for a first type of peripheral communication device and a second type of peripheral communication device peripheral communication device of the communication systems 102 according to the disclosure.

The example in FIG. 5B show a user interface 108 on a hearing protection headset 104 (e.g. see FIG. 2A) comprising three tangible controls as push-buttons 224a-224c, such as an up-button 224a a down-button 224b and a middle-button 224c, a first mapping overview 516 for at first type of peripheral communication device, being an analog radio, and a second mapping overview 518 for a second type of peripheral communication device, being a digital EUD (i.e. tactical smartphone). Both the first- 516 and second 518 mapping overview show examples of specific control-instructions 514 configured for one or more specific operation state (e.g. 508a-508c) and key combos of the tangible controls 224a-224c.

The first mapping overview 516 show that the user 100 may control the peripheral communication device 110 such as Motorola XTS 5000 radio (i.e. First type of peripheral communication device) by performing different dedicated user interactions via the user interface 108. For example, by pressing and holding the middle-button 224c in combination with long pressing the up-button 224a may cause the communication module 106 to generate a control-instruction 514 to mute or unmute (e.g. if already muted) an audio signal via a "Rx 1 line" of the receptacle interface 218 (e.g. see FIG. 4A-FIG. 4C) configured to receive audio signals from a first net or frequency band of the analog radio. The mute or unmute control-instruction may instruct the DSP 304 to gain (i.e. amplify) the analog Rx 1 line signal by -128 dB, effectually muting the audio signal or set gain to "0" to unmute). Similar, by performing the combination of by pressing and holding the middle-button 224c in combination with long pressing the down-button 224b may cause the communication module 106 to generate a control-instruction to mute or unmute an audio signal via a "Rx 2 line" of the receptacle interface 218 (e.g. see FIG. 4A-FIG. 4C) configured to receive audio signals from a second net or frequency band of the analog radio.

In case the users 100 wishes to increase or decrease the volume of the audio signals received via the Rx1 line and/or Rx2 line and emitted by the left speaker unit 212a and/or right speaker unit 212b in the headset 104 (e.g. see FIG. 2A-FIG. 2C), the user 100 may perform the combination of by pressing and holding the middle-button 224c in combination with short pressing the up-button 224a to generate a control-instruction to for increasing the volume and/or perform the combination of by pressing and holding the middle-button 224c in combination with short pressing the down-button 224b to generate a control-instruction for decreasing the volume. The volume up/down control-instructions may instruct the DSP 304 to gain the analog Rx 1 and Rx2 line signals by +2 dB/-2dB each time the control-instruction is generated (e.g. the user pressing the buttons).

If the user 100 wishes to transmit a voice signal via the transceiver module of the radio 110, the user 100 may perform the action of pressing and holding the middle-button 224c to generate a control-instruction (e.g. PTT control-instruction) applying a analog voltage level (e.g. a first control-signal) directly on the PTT line of the radio (e.g. see FIG. 4A-FIG. 4C) that switches between logic levels (typically +5V and Ground) in the transceiver module causing the radio switch from receive mode to transmit mode. The communication module 106 may additionally generate a control-instruction to the DSP 304 causing a voice signal recorded via a boom microphone 214 in the hearing protection headset 104 to be transmitted to the radio 110 via a Tx line (e.g. see FIG. 4A-FIG. 4C) such that the radio 110 may transmit the voice signal from the user 100 via the transceiver module of the radio 110 thereby establishing a person-to-person voice communication. The user 100 may switch between a first PTT1 line for the first net or frequency band of the analog radio and a second PTT2 line for the second net or frequency band of the analog radio by double pressing the middle-button 224c thereby generating a control-instruction 514 to the communication module 106 to switch the state of multiplexing (MUX) circuitry 406 to apply the analog voltage PTT signal (e.g. Control-signal) to a different connector pin (e.g. 402f in FIG. 4C ) in the receptacle interface 218 associated with the PTT2 line in the peripheral communication device 110.

The second mapping overview 419 in FIG. 5B show that the user 100 may control a digital type peripheral communication device 110 such as a Samsung Galaxy XCover6 Pro Tactical Edition (EUD) (i.e. Second type of peripheral communication device) by performing different dedicated user interactions via the user interface 108. For example, by short pressing the middle-button 224c may cause the communication module 106 to generate a control-instruction 514 to the EUD 110 as a command to answer an incoming call or perform a pause-play action on other voice media on the EUD. The answer call / pause-play control-instruction may be transmitted to the EUD according to a USB protocol sending a USB command to the EUD to perform the action. The USB command may be transmitted via the D+/D-differential pair pins (e.g. first data connector pin 404c and second data connector pin 404d see FIG. 4A-FIG. 4C). The control instruction may be communication from the communication module 106 to the EUD using a digital control-signal (e.g. Second control-signal) such as a stream of binary states (e.g. "0" and "1" bits) by for example adjusting the voltage levels above and below threshold values on one or both of the D+/D- differential pair pins (i.e. 404c and 404d in FIG. 4A-FIG. 4C.

In one embodiment, the USB command may be a co called USB HID command. A HID (Human Interface Device) command in USB protocol communication refers to a specific type of data exchange used for input devices typically structured as reports sent between the device (communication module 106) and the host (EUD 110). However not all devices or operating systems (e.g. Second (digital) type of peripheral communication devices) may interpret these HID commands identically. The effectiveness of such HID commands may depend on the specific HID descriptor used by the digital device 110 (e.g. the EUD manufacturer, Samsung, Apple etc.). Thus, the host system's (e.g. the EUD 110) may require support for these control-instructions or commands i.e. Audio HID / phone HID according to specific additional software or drivers available on the EUD 110. Advantageously, the communication system 102 may transfer the necessary drivers and/or HID command library to the host device (e.g. the EUD 110) immediately upon connection, to assure the control-instructions are supported correctly. In another advantageous embodiment, the communication system 102 may transmit digital control-instructions as the "Control transfers" according to USB protocol using predefined classes, such as "USB Audio Class" or "USB Mass Storage Class," which may be supported across different digital peripheral communications device manufacturers as default.

In the illustrated example in FIG. 5B, the user 100 may, by long-pressing the middle-button 224c cause the communication module 106 to generate a control-instruction to the EUD as a PTT over Cellular (PoC) command to perform short voice transmission via the EUD 110. The PoC control-signal may be transmitted to the EUD 110 according to a USB protocol (control-signal) sending a USB command (control-instruction) to the EUD 110 to perform the action while simultaneously sending a voice signal recorded via a boom microphone 214 in the headset 104 to be transmitted via the EUD also according to a USB protocol, such that the control-instruction to the EUD may be digital data packages containing both audio information and instructions to perform a desired function. The user 100 may increase or decrease the volume of the audio signals received via EUD and emitted by the left speaker units 212a and right speaker unit 212b in the hearing protection headset 104 (e.g. see FIG. 2A-FIG. 2C) by performing the combination of by pressing and holding the middle-button 224c in combination with short pressing the up-button 224a to generate a control-instruction for increasing the volume and/or perform the combination of by pressing and holding the middle-button 224c in combination with short pressing the down-button 224b to generate a control-instruction for decreasing the volume. The volume up/down control-signal may generate a USB command to the EUD 110 to increase or decrease the volume equivalent to using dedicated volume control buttons on the EUD 110 itself as generally known from smartphones, tablets, etc. As the EUD 110 may communicate according to a digital protocol, such as the USB protocol, the EUD may notify the communication module 106 about its current state, such as when a call is active 520 on the EUD 110 or alike, this may be handled via the State machine 360a in the system core 358 (e.g. see FIG. 4B and related description). The state of the EUD (e.g. such as "call is active 520") may cause the communication module 106 to adapt the control-instructions generated by pushing a button in response to the state of a digital type peripheral communication device 110 (e.g. such as an EUD), thus if the EUD for example is in the state "call is active 520" the control-instruction is configured for specific activation states 508a-508c of the tangible controls 224a-224c may be momentary changes automatically. For example, if the EUD state is "call is active 520" the user 100 may now perform a mute/unmute of the boom microphone 214 (or Tx signal data) by performing a short press of the middle-button 224c thus, causing the communication module 106 to generate a state dependent control-instruction as a USB commend to mute/unmute the Tx signal (e.g. boom microphone 214) on the EUD 110 device when in the "call is active 520" state. By long pressing middle-button 224c may cause the communication modules 106 to generate a control-instruction as a USB commend to end the call, thereby also interrupting the state (e.g. Call is active 520 state) of the EUD which may again automatically redefine which control-instructions the activation states are configured to perform, as previously described.

### Earcup connector interface

According to an embodiment of the disclosure, the communication system 102 for communicating in a harsh environment, comprising a hearing protection headset 104 (e.g. as described in relation to FIG. 2A-FIG. 2C and elsewhere) comprising a left connector interface 216a connected to the left earcup 200a and a right connector interface 216b connected to the right earcup 200b, wherein the left 216a and right connector interface 216b individually is configured to releasably connect a first type of accessory device and/or a second type of accessory device. The connection may be achieved via a plurality of signal pins (e.g. 606a-606c in FIG. 7B) and the communication system 102 may be configured to receive and transmit electronic signals via the plurality of signal pins (e.g. 606a-606c in FIG. 7B). Preferably, the electronic signals are in the form of data signals, more preferably in the form of audio data, such as voice data. Alternatively wherein the electronic signals are in the form of non-audio data. The communication system 102 may be configured to automatically apply and switch between a first communication mode and a second communication mode.

The first communication mode may be applied when the left connector interface 216a and/or the right connection receptacle interface 218 is/are connected to the first type of accessory device, and when in the first communication mode, the communication module enables at least a subset of the plurality of pins to receive and/or transmit one type of electronic signals, such as audio data or voice data.

The second communication mode may be applied when the left connector interface 216a and/or the right connector interface 216b is/are connected to the second type of accessory device, and, when in the second communication mode, the communication module enables at least the subset of the plurality of pins to receive and/or transmit another type of electronic signals, such as non-audio data.

In one embodiment, the first type of accessory device is a microphone device for obtaining voice data, preferably from the user when speaking, preferably wherein the microphone is attached to a flexible rod configured to be positioned close to the mouth of the user (e.g. The boom microphone 214)

In one embodiment, the second type of accessory device is a user interface, light source, a camera, a video camera or a data sensor thus, the second type of accessory device may be a toggle switch 600 providing a secondary user interface 108 to the hearing protection headsets 104 as described previously in relation to FIG. 5A and FIG. 5B.

In one embodiment, the communication module is configured to simultaneous receive and transmit electronic signals with the left connector interface and the right connector interface according to the first communication mode, when both the left and right connector interface is connected to the first type of accessory device and when in the first communication mode, the communication module enables at least a subset of the plurality of pins to receive and/or transmit one type of electronic signals, such as audio data or voice data. For example, when a boom microphone 214 may be connected to each of the left connector interface 216a and the right connector interface 216b such that two boom microphones 214 are connected to the hearing protection headset 104 simultaneously.

In one embodiment, the communication module is configured to simultaneous receive and transmit electronic signals with the left connector interface and the right connector interface according to the second communication mode, when both the left and right connector interface is connected to the second type of accessory device and when in the second communication mode, the communication module enables at least the subset of the plurality of pins to receive and/or transmit another type of electronic signals, such as non-audio data. For example, when a toggle switch 600 may be connected to each of the left connector interface 216a and the right connector interface 216b such that two toggle switches 600 are connected to the hearing protection headset 104 simultaneously.

In one embodiment, the communication module is configured to simultaneously receive and transmit electronic signals with the left connector interface according to the first communication mode, when the left connector interface is connected to the first type of accessory device and exchanging electronic signals with the right connector interface according to the second communication mode. When the right connector interface is connected to the second type of accessory device. For example, when a boom microphone 214 is connected to the left earcup 200a and a toggle switch 600 to the right earcup 200b.
or vice versa, in another embodiment, the communication module is configured to simultaneous receive and transmit electronic signals with the left connector interface according to the second communication mode, when the left connector interface is connected to the second type of accessory device and exchanging electronic signals with the right connector interface according to the first communication mode, when the right connector interface is connected to the first type of accessory device. For example, when a toggle switch 600 is connected to the left earcup 200a and a boom microphone 214 to the right earcup 200b.

FIG. 6A schematically illustrates an example of communication system 102 comprising a left connector interface 216a and a right connector interface 216b in connection with a communication module 106 according to the disclosure.

The communication module 106 (e.g. See FIG. 3A and FIG. 3B) may be in contact with the left connector interface 216a comprised by the left earcup 200a and right connector interface 216b comprised by the right earcup 200b of the hearing protection headset 104 (e.g. See FIG. 2A-FIG. 2C). Both the left connector interface 216a and the right connector interface 216b may be configured to releasably mount and connect a first type of accessory device and/or a second type of accessory. In the example in FIG. 7A the left connector interface 216a is about to releasably mount and connect the boom microphone 214 (e.g. A first type of accessory device) whereas the right connector interface 216b is about to mount and connect a toggle switch 600 (a second type of accessory device). However, it should be noted that the boom microphone 214 could also be connected to the right connector interface 216b and similar, the toggle switch 600 could also connect to the left connector interface 216a. Upon connection of the boom microphone 214 to the left connector interface 216a, the communication module 106 may be configured to receive and transmit the electronic signals, via the plurality of signal pins (e.g., see FIG. 7B) with the left connector interface 216a (e.g. And boom microphone 214) to transmit audio data as voice data of the user from the boom microphone 214 into the communication system 102. The voice data of the user of the user may be forwarded to a connected peripheral communication device 110 as described elsewhere throughout the disclosure, to be transmitted wirelessly using radio waves for example.

Additionally, upon connection of the toggle switch 600 to the right connector interface 216b, the communication module 106 may be configured receive and transmit electronic signals, via the plurality of signal pins with the right connector interface 216b (e.g. And toggle switch 600) to transmit electronic signals corresponding to operation states (e.g. Non-audio data) of the one or more tangible controls of the toggle switch 600 to the communication module 106. The communication module 106 may in response to the electronic signals corresponding to operation states generate and transmit one or control-signals and/or control-instructions to the connected peripheral communication device 110 as described in relation to FIG. 5A and elsewhere throughout the disclosure. Hence, the toggle switch 600 may provide a additional or alternative user interface for the communication system 102 relative to the user interface 108 as described in relation to FIG. 5A, FIG. 5B and elsewhere. The toggle switch 600 may comprise one tangible control that is manually operably by the user, in the form of a joystick portion 602. The tangible control element (e.g. Joystick portion 602) may support at least one operation state. The toggle switch 600 may be a 3-position switch allowing for three operation states. The user 100 may manually operate the toggle switch 600 by positioning the joystick portion 602 in different orientations, such as a first operation state may correspond to a first position 604a, a second operation state may correspond to a second position 604b and a third operation state a third position 604c. The operation states of the toggle switch 600 may generate one or more control-signals and control-instructions by the communication module 106 to a connected peripheral communication device 110. For example, the first position 604a may disable the boom microphone 214, such that not voice signal of the user may be transmitted via any connected peripheral communication devices 110 (e.g. And Interrupt any Push-to-Talk (PTT) signals), the second position 604b may generate a Push-to-Talk (PTT) signal to a first net or first talk group of a connected peripheral communication device 110 and enable VOX (e.g. Via the VOX 362b function of the communication module) such that a voice signal may be transmitted via the first net or talk group of a peripheral communication device 110 when a speak signal of the user is detected. The third position 604c may be spring-loaded meaning that the position returning back to the previous position (e.g. Second position 604b) if not activated constantly by the user 100 (e.g. Similar to the long-press state 508c of a push button 224a-224c). The third position 604c may generate a Push-to-Talk (PTT) signal to a second net or second talk group of a connected peripheral communication device 110 when activated and allow audio data from the boom microphone 214 to be transmitted to second net or second talk group of the peripheral communication device 110. This is particularly relevant when the communication system in worn by a user 100 being a combat vehicle crewman (CVC) operating in a armored military vehicle. In such as scenario, the CVC 100 may be connected to a vehicle mounted intercom system along with other crew members (e.g. Also waring respective communication systems according to the disclosure) and one ore more radios for communication outside of the vehicle. In such a configuration, may the toggle switch 600 enable a CVC 101 to communication with crem members via the intercom system when the joystick portion 602 is in the second position 604b and communication, via the radio, with externally people when the joystick portion 602 is in the third position 604c thereby providing clear and undistorted communication for CVC 100 operating in harsh and demanding environments.

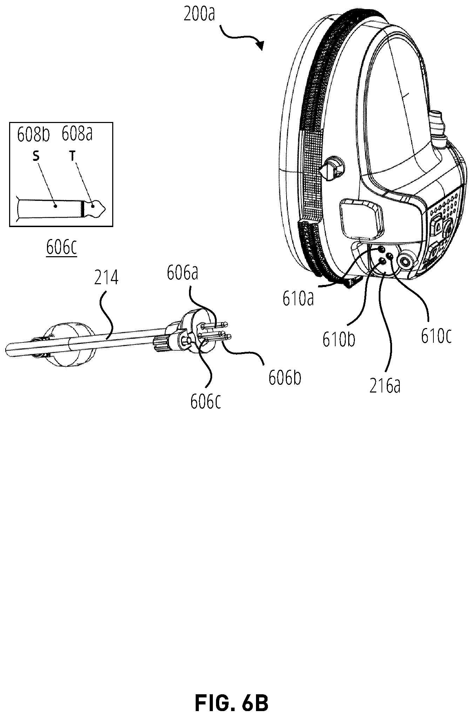

FIG. 6B schematically illustrates an example of a left earcup 200a of a hearing protection headset 104 comprising a left connector interface 216a and a first type of accessory device according to the disclosure. In the example in FIG. 6B the first type of accessory device being a boom microphone 214 (e.g. See FIG. 2A) comprising a first signal pin 606a, a second signal pin 606b and a sense signal pin 606c. The left receptacle interface 218 of the left earcup 200a comprise a first signal terminal 610a, a second signal terminal 610b and a sense signal terminal 610c. The terminal 610a-610c may be configured to releasably connect with respective pins (606a-606c) to make a physical and electrical connection, such that electronic signals may be exchanged (e.g. receive and transmit) between the communication system 102 comprising the communication module 106 and left connector interface 216a.

In one embodiment, at least the sense signal pin 606c may be a TS jack type pin. The TS (Tip-Sleeve) jack type signal pin 606c allows two separate electrical contacts to be contained within the formfactor of a single physical pin. This is advantageous as it reduces the total number of physical connector pins to 3 allowing the physical embodiment of the plug in an asymmetric manner such that the user may only fit the plug into the socket in one (correct) orientation.

In some embodiments at least the sense pin may be a TRS or TRRS jack type pin. The TRS (Tip-Ring-Sleeve) jack type connector pin allow tree separate electrical contacts, whereas the TRRS (Tip-Ring-Ring-Sleeve) jack type connector pin allow four separate electrical contacts with in the formfactor of a single physical pin.

In the present example in FIG. 6B, a sub-window shows the sense signal pin 606c in a configuration where the single physical pin provide two separate electrical contact points, namely a "T" tip contact 608a and a "S" sleeve contact 608b. The corresponding sense signal terminal 610c may support the tip contact 608a via a tip terminal 612a and the sleeve contact 608b via a sleeve terminal 612b (e.g. See FIG. 6C). The sense signal pin 606c may in other variations be a single contact pin. Although the illustration in FIG. 6B show an example of the arrangement seen from the left earcup 200a of the hearing protection headset 104 and a first type of accessory device (e.g. The boom microphone 214), it should be noted that a similar arrangement may be used to describe the right earcup 200b of the hearing protection headset 104, the right connector interface 216b and the signal pins for a second type of accessory device.

In an alternative embodiment, the left connector interfaces 216a and right connector interface 216b may communication wirelessly with at least a first accessory (e.g. A boom microphone 214) device using inductive coupling. Wherein, the left connector interface 216a and/or right connector interface 216b may exchange electronic signals and/or energy from at least one circuit to another through a shared magnetic field.

FIG. 6C schematically illustrates an example of the communication module 106 configured to to automatically apply and switch between exchanging the electronic signals with the left connector interface 216a according to a first communication mode and a second communication mode. The communication module 106 may comprise a switching circuitry 616 having a first switch 616a and second switch 616b in electrical connection with the left connector interface 216a. Each of the switches 616a, 616b may be arranged in a first state 410 or a second state 412 thereby altering the signal routing between the left connector interface 216a and the communication module. The switching circuitry 616 may not be a actual physical switch or relay component but rather represent a visual representation of a way to alter the active connection lines between the left connector interface 216a and the communication module 106 such that the exchange of electronic signals may be switched between at least the first communication mode and the second communication mode. In the present example in FIG. 6C, the left connector interface 216a is connected to a boom microphone 214 (e.g. First type of accessory device) via the first terminal 402a in electrical contact with the first signal pin 606a, the second terminal 402b in electrical contact with the second signal pin 606b and the sense signal terminal 610c in contact with the sense signal pin 606c. In the example, the sense signal pin 606c is a TS jack type pin allowing for a tip contact 608a to be in electrical contact with a tip terminal 612a part of the sense signal terminal 610c and a sleeve contact 608b to be in electrical contact with a sleeve terminal 612b part of the sense signal terminal 610c. The sleeve contact 608b and sleeve terminal 612b may be connected to a common electrical ground. The tip contact 608a may be connected to a sense chip 614 circuitry of the boom microphone 214 used for providing identification data to the communication module 106. The tip terminal 612a may be connected to the accessory module 356e of the communication module 106 configured to obtain the identification data from the boom microphone 214 such as via the sense chip 614 in a similar manner as described in relation to coupling data elsewhere though out the disclosure. However, in a more simple scenario, the sense chip 614 may be a 10 kΩ resistor connecting the first signal pin 606a and the sense signal pins 606c (e.g. Where the sense signal pin 606c being a simple single contact type pin), such that identification data may be obtained by reading a specific voltage level measured by the communication module 106 via the sense signal terminal 610c (e.g. Connected to ground) relative to the first terminal 402a and thereby identifying the type of accessory device, for example via a look-up table scored in memory 306 of the communication module 106. In response to the obtained identification data, the communication module 106 may automatically adjust the first switch 616a and second switch 616b, thereby establishing communication via a first communication mode or a second communication mode. In the present example the communication module 106 may recognize the boom microphone 214 as a first type of accessory device (e.g. Via first identification data) and automatically adjust the signal routing by setting both the first switch 616a and the second switch 616b in their first state 410 (as indicated on FIG. 6C as a black arrow) such that the communication module 106 (e.g. Via the CODEC module 348) may receive and transmit electronic signals with the first signal pin 606a and the second signal pin 606b in the form of audio data, such as voice data from the user 100. In case the first type of accessory device is a analog microphone unit, voice data from the user 100 may be transmitted from the boom microphone 214 to the hearing protection headset 104 comprising the communication module 106, using the first signal pin 606a and second signal pin 606b according to "balanced signaling" where the two conductors that have equal impedance to ground and/or differential signaling where the two conductors transmitting the same signal at opposite polarity. A balanced signal line may often utilize differential signaling but not always. A balanced line may have one conductor (e.g. 606a) with an audio signal, and the other (e.g. 606b) with no signal (0V) or each conductor (e.g. 606a and 606b) may carry the same signal at opposite polarity (differential). For a balanced line both conductors (e.g. 606a, 606b) have the same impedance to ground (e.g. 612b). In case the first type pf accessory device is a digital microphone unit, such as a USB microphone device, voice data from the user 100 may be transmitted from the boom microphone 214 to the hearing protection headset 104 comprising the communication module 106, using the first signal pin 606a and second signal pin 606b as the D+/D- differential pair according the USB communication protocol for digital USB audio data, as describe in details elsewhere.

In a another example, the left connector interface 216a may connect to second type of accessory device. In this case, the accessory module 356e may obtain second type of identification data, thereby recognizing that the connected second type of accessory device as a toggle switch 600 causing the communication module 106 to automatically adjust the first switch 616a and second switch 616b into their second state 412 (e.g. Along the dotted line in FIG. 6C). This may enable the communication module 106 to exchange electronic signals with the first signal terminal 610a and second terminal 402b and corresponding pins of the toggle switch 600 (e.g. First signal pin and second signal pin of the second type of accessory device) according to the second communication mode. In the second communication mode, the electronic signals may comprise non-audio data. The communication module 106 may communication with the first signal pin of the toggle switch 600 using a 1-way digital communication protocol such as UART via the UART module 356m, such that low bit rate communication signals may be exchanged, enabling the toggle switch 600 to electronic signals related to the operation states (e.g. Positions 604a-604c) of the joystick portion 602. Additionally, the power manager 312 may be contact with the second signal pin of the second type of accessory device. In yet another example, the second type of accessory device may be a light source configured to emit light, such that the user 100 may be able to read a map in darkness or alike. The light source may comprise one or more Light Emitting Diodes (LED) elements for providing illumination. The light sources may be an RGB light source, comprising individual red (around 630 nm), green (around 532 nm) and blue (around 465 nm) LEDs and or a (infra-red) IR light source (e.g. Spectrum: 800 nm - 2000 nm). The communication module 106 may recognize the second type of accessory device as being a light source based on identification data (e.g. Second identification data) and adjust the second switch 616b such the communication module 106 may receive and transmit electronic signals with the light source according to the second communication mode. Thus, the power managers 312 may be enabled to deliver power to the second signal pin for powering the one or more LEDs of the light source. Additionally, the first signal pin of the light source may be configured to communication 1-way UART using the UART function 356m via the first signal pin of the light source, such as to support a button or knob on the light source for toggling between illumination modes (e.g. ON/OFF, IR, R, G, B or white) or adjust the light intensity via the power input from the power manager 312 to the light source.

Although the illustration in FIG. 6C show an example of the arrangement seen from the left earcup 200a of the hearing protection headset 104, it should be noted that a similar arrangement may be used to describe how the communication module 106 may be configured to automatically apply and switch between receive and transmit the electronic signals according to least a first communication mode and a second communication mode with the right connector interface 216b, when connected to a first or second type of accessory device (e.g. 600, 214).

In one embodiment, the voice data of the user comprises analog audio signals and/or digital audio data packages, preferably according to a USB protocol, a UART protocol, or an EtherNet/IP protocol, when the communication module 106 is exchanging the electronic signals with the left connector interface 216a and or right connector interface 216b according to the first communication mode.

In one embodiment, non-audio data comprise analog non-audio signals and/or digital non-audio data packages, preferably according to USB protocol, a UART protocol, or an EtherNet/IP protocol, such as video data, control instructions, commands, instructions, metadata. GPS coordinates, images etc., when the communication module 106 is exchanging the electronic signals with the with the left connector interface 216a and or right connector interface 216b according to the second communication mode.

### Configurations

Some exemplary embodiments of the communication system 102 and different types connected peripheral communication devices 110 are provided in FIG. 7A-FIG. 7C in accordance with at least some embodiments of the disclosure.

FIG. 7A schematically illustrate a first configuration 702 of a communication system 102 according to the disclosure, embodied in a circumaural hearing protection headset 104 to be worn by a user 100 as described in relation to FIG. 1, FIG. 2A-FIG. 2C and elsewhere.

In the first configuration 702, the hearing protection headset 104 is connected to a peripheral communication device 110 via a cable 112 and a connector 220 (e.g. plug) as described in detail elsewhere. The connected peripheral communication device 110 in the first configuration 702 being a Motorola XTS 5000 radio 708 worn by the user 100 in harsh and demanding environments (e.g. a first type of peripheral communication device). The radio 708 may be connected directly to the headset 104 comprising the communication module 106 (e.g. as described in relation to FIG. 3A-FIG. 3B) via an electronic receptacle interface 218 (e.g. socket) as described in relation to FIG. 4A-FIG. 4C and elsewhere configured to recognize the type of device 110 being connected to the electronic receptacle interface 218 (e.g. socket). The communication module 106 may receive first coupling data when in contact with the first type of peripheral communication device 708 (e.g. via a cable chip 222), specifying the radio 708 is an analog communication device type configured to communicate with the hearing protection headset 104 using analog control- and audio signals. Thus, the headset 104 may set the one or more switching circuits 408a-408d of the multiplexing (MUX) circuitry 406 such that the plurality of connector pins 404a-404g are configured in a configuration enabling exchange of electronic signals in according to the first data exchange mode as illustrated in FIG. 4C, allowing analog PTT control-signals to be transmitted via the PTT connector pin 404e to the transceiver module of the radio 708 to enable person-to-person voice communication. The audio transmission connector pin 404b may be configured to transmit audio data from the hearing protection headset 104 to the radio 708 (e.g. voice data recorded via the boom microphone 214 to be send to the transceiver module of the radio 708). Additionally, the first data connector pin 404c and the second data connector pin 404d are configured solely to receive audio data from the 708, such that RF voice communication data received by the transceiver module of the radio 708 may be directed to the headset 104 to be played to the user 100 via the left speaker unit 212a and/or right speaker unit 212b of the headset 104. The coupling data may additionally specify how the user interfaces 108 of the communication systems 102 should respond according to manual interactions by the user 100 as described in relation to FIG. 5A, FIG. 5B and elsewhere. The user interface 108 of the headset 104 may thus cause the communication module 106 to generate one or more control-instructions as described in relation to the first mapping overviews 516 displayed in FIG. 5B and elsewhere.

The coupling data obtained when connecting the Motorola XTS 5000 radio 708 with the hearing protection headset 104 may specify a "power mode" to be "from radio" meaning that the hearing protection headset 104 may rely on an external power source for powering the communication system. Thus, the multiplexing (MUX) circuitry 406 may configure the sixth terminal 402f and corresponding power connector pin 404f in a configuration allowing the communication system to draw parasitic power from the battery of the Motorola XTS 5000 radio 708.

FIG. 7B schematically illustrates a second configuration 704 of a communication system 102 according to the disclosure, embodied in a circumaural hearing protection headset 104 to be worn by a user 100 as described in relation to FIG. 1, FIG. 2A-FIG. 2C and elsewhere.

In the second configuration 704, the hearing protection headset 104 is connected to a peripheral communication device 110 via a cable 112 and a connector 220 (e.g. plug) as described in detail elsewhere. The connected peripheral communication device 110 in the second configuration 704 being a chest mounted Samsung Galaxy XCover6 Pro Tactical Edition EUD 710 worn by the user 100 in harsh and demanding environments (e.g. a second type of peripheral communication device). The EUD 710 may be connected directly to the headset 104 comprising the communication module 106 (e.g. as described in relation to FIG. 3A and FIG. 3B) via an electronic receptacle interface 218 (e.g. socket) as described in relation to FIG. 4A-FIG. 4C and elsewhere configured to recognize the type of device being connected to the electronic receptacle interfaces 218 (e.g. socket). The communication module 106 may receive second coupling data when in contact with the second type of peripheral communication device 710 (e.g. via a cable chip 222) specifying that the EUD 710 is a digital communication device type configured to communicate using digital control- and audio signals with the communication system 102, (e.g. using a USB protocol). Thus, the headset 104 may set the one or more switching circuits 408a-408c of the multiplexing (MUX) circuitry 406 such that the plurality of connector pins 404a-404g are configured in a configuration enabling exchange of electronic signals in according to the second data exchange mode, where the first data connector pin 404c and the second data connector pin 404d are configured for exchanging both audio data and non-audio data between the 710 and the headset 104 as described in detail in relation to FIG. 4C. Thus, the third terminal 402c and the fourth terminal 402d of the receptacle interface 218 may be configured as the USB differential transmission pair for data (D+ and D-) for exchange of Digital data packages (e.g. USB transaction). The coupling data may additionally specify how the user interface 108 of the communication system 102 should respond according to manual interactions by the user 100 as described in relation to FIG. 5A, FIG. 5B and elsewhere. The user interface 108 of the headset 104 may thus cause the communication module 106 to generate control-instructions as described in relation to the second mapping overview 518 displayed in FIG. 5B and elsewhere.

The coupling data obtained when connecting the Samsung Galaxy XCover6 Pro Tactical Edition EUD 710 with the hearing protection headset 104 may specify a "power mode" to be "battery" meaning that the hearing protection headset 104 may utilize the internal battery as the main power source for powering the communication system. However, as the EUD 710 is communicating using a digital USB protocol, the sixth terminal 402f and corresponding power connector pin 404f may be configured such that the Samsung Galaxy XCover6 Pro Tactical Edition EUD 710 is providing the necessary voltage to power the USB bus of the communication system 102 via a inbuild Li-Ion battery in the device.

FIG. 7C schematically illustrate a third configuration 706 of a communication system 102 according to the disclosure, embodied in a circumaural hearing protection headset 104 to be worn by a user 100 as described in relation to FIG. 1, FIG. 2A-FIG. 2C and elsewhere.

In the third configuration 706, the hearing protection headset 104 is connected to a third peripheral communication device 110 via a cable 112 and a connector 220 (e.g. plug) as described in detail elsewhere. The connected peripheral communication device in the third configuration 706 being a west worn PTT control unit 712 (e.g. a third type of connected peripheral communication device). The PTT control unit 712 may be communication hub, such as the INVISIO V60 ADP control unit, connected to a Persistent systems, Persistent systems wave relay MPUS 5 radio 714 and a Thales AN/PRC-148 JEM radio 716. The PTT control unit 712 may additionally comprise a communication module 106 and one or more electronic receptacle interfaces 218 as described herein, for communication, audio and data exchange with the connected radios 714, 716 and headset 104. The PTT control unit 712 may be connected directly to the hearing protection headset 104 comprising the communication module 106 (e.g. as described in relation to FIG. 3A, FIG. 3B) via an electronic receptacle interface 218 (e.g. socket) as described in relation to FIG. 4A-FIG. 4C and elsewhere configured to recognize the type of device being connected to the electronic receptacle interface 218 (e.g. socket). The communication module 106 may receive third coupling data when in contact with the third type of peripheral communication device 712 (e.g. via a cable chip 222), specifying that the PTT control unit 712 is a hybrid device type configured to exchange audio data according to analog signals and control-instructions using digital data packages according to a digital communication protocol. Thus, the headset 104 may set the one or more switching circuits 408a-408d of the multiplexing (MUX) circuitry 406 such that the plurality of connector pins 404a-404g are configured in a configuration where the audio transmission connector pin 404b is configured to transmitting audio data from the headset 104 to the PTT control unit 712 (e.g. voice data recorded via the boom microphone 214 to be send to the transceiver module of one of the 714, 716 via the PTT control unit 712). Additionally, the first data connector pin 404c and the second data connector pin 404d are configured, according to the first data exchange mode, solely to receive audio data from the PTT control unit 712, such that for example RF voice communication data received by the transceiver module of the Persistent systems wave relay MPUS 5 radio 714 may be directed to the hearing protection headset 104 via the first data connector pin 404c to be played to the user 100 via the right left speaker unit 212a and/or right speaker unit 212b of the headset 104 and RF voice communication data received by the transceiver module of the Thales AN/PRC-148 JEM radio 716 may be directed to the hearing protection headset 104 via the second data connector pin 404d to be played to the user 100 via the left speaker unit 212a/or right speaker unit 212b of the hearing protection headset 104. The third switching circuit 408c of the communication module may be set (i.e. to the first state, along the dashed line in FIG. 4C) such that the fifth terminal 402e enables the hearing protection headset 104 to exchange digital data packages with the PTT control unit 712 via the UART interface using a half-duplex or one-wire UART data exchange protocol. Thus enables the headset 104 and PTT control unit 712 to exchange control-instructions as digital data packages. The PTT control unit 712 may additionally comprise a user interface comprising a plurality of tangible controls for controlling functions of PTT control unit 712 itself and its connected communication devices (e.g. radio 714 and 716). The coupling data may additionally specify how the user interface 108 of the 104 should respond according to manual interactions by the user 100 as described in relation to FIG. 5B and elsewhere. The user interface 108 of the headset 104 may thus cause the communication module 106 to generate control-instructions according to the UART communication protocol for exchanging information with the PTT control unit 712 to for example control the Persistent systems, Persistent systems wave relay MPUS 5 radio 714 and/or the Thales AN/PRC-148 JEM radio 716 by generating a control-instruction such as a UART PTT event call from the one or more tangible controls 224a-224c to perform an action in the PTT control unit 712 equivalent by pressing one or more buttons on the PTT control unit 712 itself or vice versa, such that button functionality are mirrored between the user interface 108 of the headset 104 and the user interface of the PTT control unit 712 providing easy and intuitive operation for the user 100 as the radios 714 and 716 may be operated via multiple synchronized user interfaces providing the same operations.

Some preferred embodiments have been shown in the foregoing, but it should be stressed that the invention is not limited to these, but may be embodied in other ways within the subject matter defined in the following claims.

It should be emphasized that the term "comprises/comprising" when used in this specification is taken to specify the presence of stated features, elements, steps or components but does not preclude the presence or addition of one or more other features, elements, steps, components or groups thereof.

In the claims enumerating several features, some or all of these features may be embodied by one and the same element, component or item. The mere fact that certain measures are recited in mutually different dependent claims or described in different embodiments does not indicate that a combination of these measures cannot be used to advantage.

In the claims, any reference signs placed between parentheses shall not be constructed as limiting the claim. The word "comprising" does not exclude the presence of elements or steps other than those listed in a claim. The word "a" or "an" preceding an element does not exclude the presence of a plurality of such elements.

The mere fact that certain measures are recited in mutually different dependent claims does not indicate that a combination of these measures cannot be used to an advantage.

It will be apparent to a person skilled in the art that the various embodiments of the invention as disclosed and/or elements thereof can be combined without departing from the scope of the invention as defined in the claims.

## Claims

1. A communication system (102) for communicating in a harsh environment, comprising:
- a hearing protection headset (104) to be worn by a user (100) in the harsh environment, said headset comprising:
- a left earcup (200a) and a right earcup (200b) configured to attenuate external sounds from the harsh environment;
- a communication module (106) configured to:
- connect with a first type of peripheral communication device (708) and a second type of peripheral communication device (710),
- receive at least one audio signal from the first type of peripheral communication device and/or the second type of peripheral communication device,
- transmit the at least one audio signal to a speaker (212a) in the left earcup (200a) and/or a speaker (212b) in the right earcup (200b),
- transmit a first control-signal comprising one or more first control instruction(s) of and to the first type of peripheral communication device, and
- transmit a second control-signal comprising one or more second control instruction(s) of and to the second type of peripheral communication device, wherein the first control-signal is a first type of signal, and the second control-signal is a second type of signal that differs from the first type of signal; and
- a user interface (108) in connection with the communication module (106), wherein the user interface (108) comprises one or more tangible controls (224a, 224b, 224c) that are manually operably by the user, wherein each of said tangible controls has at least one operation state, wherein each operation state is automatically configured to enable the communication module (106) to:
- transmit one or more control-instruction(s) of said first control-instruction(s) using the first control-signal; or
- transmit one or more control-instruction(s) of said second control-instruction(s) using the second control-signal.

2. The communication system (102) according to claim 1, wherein each operation state is automatically configured for each of the tangible controls to enable the communication module (106) to:
- transmit the one or more control-instruction(s) of said first control-instruction(s) using the first control-signal, when the communication module receives first coupling data associated with the first type of peripheral communication device, or
- transmit the one or more control-instruction(s) of said second control-instruction(s) of the second type of peripheral communication device using the second control-signal when the communication module (106) receives second coupling data associated with the second type of peripheral communication device.

3. The communication system (102) according to claim 2, wherein the communication module (106) is configured to receive the first coupling data when the communication module (106) is in contact with the first type of peripheral communication device, and/or wherein the communication module (106) is configured to receive the second coupling data when the communication module (106) is in contact with second type of peripheral communication device.

4. The communication system (102) according to claim 3, wherein the communication module (106) is further configured to receive the first coupling data and/or second coupling data from a socket (218) located in the communication module (106) and/or from a plug (220) being connected to the communication modules (106), preferably where the socket (218) is configured to recognize the type of device being connected to the socket (218), more preferably where the plug (220) comprises a microchip (222) comprising the first coupling data or the second coupling data.

5. The communication system (102) according to any one of claims 2 to 4, wherein the first coupling data comprises said first control-instruction(s), and/or wherein the second coupling data comprises said second control-instruction(s).

6. The communication system (102) according to any of the preceding claims, wherein the one or more control-instructions of said first control-instruction(s) is defined according to a first communication protocol or wherein the one or more control-instructions of said second control-instruction(s) is defined according to a second communication protocol.

7. The communication system (102) according to any of the preceding claims, wherein the one or more control-instructions of said first control-instruction(s) is defined to control a transceiver-module of the first type of peripheral communication device to establish a person-to-person voice communication, and/or wherein the one or more control-instructions of said second control-instruction(s) is defined to control a transceiver-module of the second type of peripheral communication device to establish a person-to-person voice communication.

8. The communication system (102) according to any of the preceding claims, wherein the first control-signal is a digital signal and wherein the second control-signal is an analog signal, or wherein the first control-signal is an analog signal and wherein the second control-signal is a digital signal.

9. The communication system (102) according to claim 8, wherein the analog signal comprises a Push-to-Talk (PTT) signal, preferably where the PTT signal is a DC voltage signal or an AC voltage signal and/or wherein the digital signal comprises digital data packages, preferably where the digital signal is a USB signal, a UART signal, an ethernet signal or a DECT signal.

10. The communication system (102) according to any of the preceding claims, wherein the one or more tangible controls is/are in the form of a one or more physical buttons (224a, 224b, 224c) or switches.

11. The communication system (102) according to any of the preceding claims, wherein the at least one operation state of each of said tangible controls is one of:
- a short-press state, wherein the short-press state is defined as an interaction with the tactile control (224a, 224b, 224c) for less than 200 ms, and
- a long-press state, wherein the long-press state is defined as an interaction with the tactile control (224a, 224b, 224c) for more than 250 ms.

12. The communication system (102) according to any of the preceding claims, wherein the first and/or second type of peripheral communication device is one or more of a chest mounted tactical EUD, a Military LMR, a vehicle mounted intercom system, and a vest worn PTT control unit.

13. The communication system (102) according to any of the preceding claims, wherein the communication module (106) and the user interface (108) are integrated into a single communication device that is located separately from the hearing protection headset or wherein the single communication device is integrated into a single communication device located in the hearing protection headset (104) or located in a head-worn device comprising the hearing protection headset (104).

14. A communication device for a communication system (102) for communicating in a harsh environment, the communication device comprising:
- a communication module (106) configured to:
- connect with a first type of peripheral communication device and a second type of peripheral communication device,
- receive at least one audio signal from the first type of peripheral communication device and/or the second type of peripheral communication device,
- transmit the at least one audio signal to a speaker (212a) in a left earcup (200a) of a headset (104) and/or a speaker (212b) in a right earcup (200b) of a headset (104),
- transmit a first control-signal comprising one or more first control instruction(s) of and to the first type of peripheral communication device, and
- transmit a second control-signal comprising one or more second control instruction(s) of and to the second type of peripheral communication device, wherein the first control-signal is a first type of signal, and the second control-signal is a second type of signal that differs from the first type of signal; and
- a user interface in contact with the communication module (106), wherein the user interface (108) comprises one or more tangible controls (224a, 224b, 224c) that are manually operably by the user, wherein each of said tangible controls (224a, 224b, 224c) has at least one operation state, wherein each operation state is automatically configured to enable the communication module (106) to:
- transmit one or more control instructions of said first control-instruction(s) using the first control-signal; and/or
- transmit one or more control-instructions of said second control-instruction(s) using the second control-signal.

15. The communication device according to claim 14, wherein the communication device is for the communication system according to any of the claims 1-13.
